# EUROPEAN PATENT SPECIFICATION

(11) **EP 3 087 197 B1**
(45) Date of publication and mention of the grant of the patent: **10.10.2018**
(21) Application number: 14818949.1
(22) Date of filing: 17.12.2014
(51) Int. Cl.: C12Q 1/68

(54) **DETECTION METHODS FOR SAMPLE SPECIES ORIGIN**
ERKENNUNGSVERFAHREN FÜR SPECIES URSPRUNG VON PROBEN
PROCÉDÉS DE DÉTECTION DE L'ORIGINE EN TERME D'ESPÈCE DANS UN ECHANTILLON A TESTER

(30) Priority: 27.12.2013 EP 13199634
(43) Date of publication of application: 02.11.2016
(73) Proprietor: Université de Liège, 4031 Angleur (BE); Quality Partner S.A., 4040 Herstal (BE)
(72) Inventor: DAUBE, Georges, B-4000 Liège (BE); BURTEAU, Sophie, B-4000 Liège (BE); NEZER, Carine, B-4040 Herstal (BE); DELHALLE, Laurent, B-4040 Herstal (BE)
(74) Representative: Barker Brettell LLP
(86) International application number: PCT/EP2014/078179
(87) International publication number: WO 2015/097030

(56) References cited:
- WO-A1-2011/023950
- WO-A2-2012/056227
- US-A1- 2006 099 617

### SEQUENCE LISTING

<110> Université de Liège and Quality Partner s.a.
<120> Detection Methods
<130> 2013-55
<150> EP13199634.0
   <151> 2013-12-27
<160> 103
<170> PatentIn version 3.5
<210> 1
   <211> 21
   <212> DNA
   <213> Artificial Sequence
<220>
   <223> Primer
<400> 1
   agtctatgtt tatatttacc t 21
<210> 2
   <211> 21
   <212> DNA
   <213> Artificial Sequence
<220>
   <223> Primer
<400> 2
   aatctytgtt tatatttacc t 21
<210> 3
   <211> 20
   <212> DNA
   <213> Artificial Sequence
<220>
   <223> Primer
<400> 3
   tcatcrtctt ccaargagcc 20
<210> 4
   <211> 51
   <212> DNA
   <213> Artificial Sequence
<220>
   <223> Primer
<400> 4
   cctatcccct gtgtgccttg gcagtctcag agtctatgtt tatatttacc t 51
<210> 5
   <211> 51
   <212> DNA
   <213> Artificial Sequence
<220>
   <223> Primer
<400> 5
   cctatcccct gtgtgccttg gcagtctcag aatctytgtt tatatttacc t 51
<210> 6
   <211> 59
   <212> DNA
   <213> Artificial Sequence
<220>
   <223> Primer
<400> 6
   ccatctcatc cctgcgtgtc tccgactcag agactgcact catcrtcttc caargagcc 59
<210> 7
   <211> 59
   <212> DNA
   <213> Artificial Sequence
<220>
   <223> Primer
<400> 7
   ccatctcatc cctgcgtgtc tccgactcag atcgcaggct catcrtcttc caargagcc 59
<210> 8
   <211> 60
   <212> DNA
   <213> Artificial Sequence
<220>
   <223> Primer
<400> 8
   ccatctcatc cctgcgtgtc tccgactcag acgctcgaca tcatcrtctt ccaargagcc 60
<210> 9
   <211> 60
   <212> DNA
   <213> Artificial Sequence
<220>
   <223> Primer
<400> 9
   ccatctcatc cctgcgtgtc tccgactcag agacgcactc tcatcrtctt ccaargagcc 60
<210> 10
   <211> 60
   <212> DNA
   <213> Artificial Sequence
<220>
   <223> Primer
<400> 10
   ccatctcatc cctgcgtgtc tccgactcag agcactgtag tcatcrtctt ccaargagcc 60
<210> 11
   <211> 60
   <212> DNA
   <213> Artificial Sequence
<220>
   <223> Primer
<400> 11
   ccatctcatc cctgcgtgtc tccgactcag atcagacacg tcatcrtctt ccaargagcc 60
<210> 12
   <211> 60
   <212> DNA
   <213> Artificial Sequence
<220>
   <223> Primer
<400> 12
   ccatctcatc cctgcgtgtc tccgactcag atatcgcgag tcatcrtctt ccaargagcc 60
<210> 13
   <211> 60
   <212> DNA
   <213> Artificial Sequence
<220>
   <223> Primer
<400> 13
   ccatctcatc cctgcgtgtc tccgactcag cgtgtctcta tcatcrtctt ccaargagcc 60
<210> 14
   <211> 27
   <212> DNA
   <213> Artificial Sequence
<220>
   <223> Primer
<400> 14
   gctagcagtc tatgtttata tttacct 27
<210> 15
   <211> 27
   <212> DNA
   <213> Artificial Sequence
<220>
   <223> Primer
<400> 15
   actgcaaatc tytgtttata tttacct 27
<210> 16
   <211> 20
   <212> DNA
   <213> Artificial Sequence
<220>
   <223> Primer
<400> 16
   tcatcrtctt ccaargagcc 20
<210> 17
   <211> 18
   <212> DNA
   <213> Sus scrofa
<400> 17
   ctgattgttg ctggtccc 18
<210> 18
   <211> 18
   <212> DNA
   <213> Sus scrofa
<400> 18
   gtttccgtcg tagcgtga 18
<210> 19
   <211> 25
   <212> DNA
   <213> Sus scrofa
<400> 19
   tgtaatgcat gtatgtggag acaaa 25
<210> 20
   <211> 27
   <212> DNA
   <213> Gallus gallus
<400> 20
   gctagcagtc tatgtttata tttacct 27
<210> 21
   <211> 23
   <212> DNA
   <213> Gallus gallus
<400> 21
   ttcatgcaga tcgcrgttga tcc 23
<210> 22
   <211> 27
   <212> DNA
   <213> Bos taurus
<400> 22
   actgcaaatc tctgtttata tttacct 27
<210> 23
   <211> 29
   <212> DNA
   <213> Bos taurus
<400> 23
   tgtttgtgga gggaaaacac tacatcctc 29
<210> 24
   <211> 27
   <212> DNA
   <213> Ovis aries
<400> 24
   actgcaaatc tttgtttata tttacct 27
<210> 25
   <211> 30
   <212> DNA
   <213> Ovis aries
<400> 25
   catgcttgtg gagacaaaac aataaatcct 30
<210> 26
   <211> 29
   <212> DNA
   <213> Equus caballus
<400> 26
   gcaaatctct gtttatattt acctgtttg 29
<210> 27
   <211> 22
   <212> DNA
   <213> Equus caballus
<400> 27
   ggtaatgatt gtttccgtcg tc 22
<210> 28
   <211> 24
   <212> DNA
   <213> Equus caballus
<400> 28
   ttcttgctgg tccagtggat ctaa 24
<210> 29
   <211> 27
   <212> DNA
   <213> Meleagris gallopavo
<400> 29
   gctagcagtc tatgtttata tttacct 27
<210> 30
   <211> 22
   <212> DNA
   <213> Meleagris gallopavo
<400> 30
   tgcagatttt agttcatccg gt 22
<210> 31
   <211> 15
   <212> DNA
   <213> Artificial Sequence
<220>
   <223> Primer
<400> 31
   ggaactgatt gatca 15
<210> 32
   <211> 23
   <212> DNA
   <213> Artificial Sequence
<220>
   <223> Primer
<400> 32
   agtggaggag cyttgggyaa aag 23
<210> 33
   <211> 1128
   <212> DNA
   <213> Coturnix chinensis
<400> 33
   atgcaaaagc tagcagtcta tgtttatatt tacctgttcg tgcagatatc tgttgatccg      60
   gtggctctcg atggcagtag tcagcccaca gagaacactg aaaaagacgg actgtgcaat     120
   gcttgtacgt ggagacagaa cacaaaatcc tccagaatag aagccataaa aattcaaatc     180
   ctcagcaaac tgcgcctgga acaagcacct aacattagca gggacgttat taaacaactt     240
   ctacccaaag ctcctccact gcaggaactg attgatcagt acgacgtcca gagagatgac     300
   agtagcgatg gctctttgga agatgatgac tatcatgcca caaccgaaac gattatcaca     360
   atgcctacgg agtctgattt tcttgtacaa atggagggaa aaccaaaatg ttgcttcttt     420
   aagtttagct ctaagataca atataacaaa gtagtaaagg cacaattatg gatatacttg     480
   aggcaagtcc aaaaacctac aacggtgttt gtgcagatcc tgagactcat taaacccatg     540
   aaagacggta caagatatac tggaattcga tctttgaaac ttgacatgaa cccaggcaat     600
   ggtatctggc agagtattga tgtgaagaca gtcttgcaaa attggctcaa acagcctgaa     660
   tccaacttag gcatcgaaat aaaagctttt gatgagaatg gacgagatct tgctgtaaca     720
   ttcccaggac caggtgaaga cggatcgaac ccatttttag aggtcagagt tacagataca     780
   ccaaaacggt cccgcagaga ttttggcctt gactgtgatg agcactcaac agaatctcga     840
   tgttgtcgct accccttgac ggtggatttt gaagcttttg gatgggactg gattatagcg     900
   cctaaaagat acaaagcaaa ttactgctct ggagaatgcg aatttgtatt tctacagaaa     960
   taccctcaca ctcacctggt gcaccaagca aatccaagag gttcagcagg cccttgctgc    1020
   acacccacca agatgtcccc tataaatatg ctgtatttca atggaaaaga acaaataata    1080
   tatggaaaga taccagctat ggttgtagat cgttgcgggt gctcatga                 1128
<210> 34
   <211> 1128
   <212> DNA
   <213> Gallus gallus
<400> 34
   atgcaaaagc tagcagtcta tgtttatatt tacctgttca tgcagatcgc ggttgatcca      60
   gtggctctgg atggcagtag tcagcccaca gagaacgctg aaaaagacgg actgtgcaat     120
   gcttgtacgt ggagacagaa tacaaaatcc tccagaatag aagccataaa aattcaaatc     180
   ctcagcaaac tgcgcctgga acaagcacct aacattagca gggacgttat taagcagctt     240
   ttacccaaag ctcctccact gcaggaactg attgatcagt atgatgtcca gagggacgac     300
   agtagcgatg gctctttgga agacgatgac tatcatgcca caaccgagac gattatcaca     360
   atgcctacgg agtctgattt tcttgtacaa atggagggaa aaccaaaatg ttgcttcttt     420
   aagtttagct ctaaaataca atataacaaa gtagtaaagg cacaattatg gatatacttg     480
   aggcaagtcc aaaaacctac aacggtgttt gtgcagatcc tgagactcat taagcccatg     540
   aaagacggta caagatatac tggaattcga tctttgaaac ttgacatgaa cccaggcact     600
   ggtatctggc agagtattga tgtgaagaca gtgctgcaaa attggctcaa acagcctgaa     660
   tccaatttag gcatcgaaat aaaagctttt gatgagactg gacgagatct tgctgtcaca     720
   ttcccaggac cgggtgaaga tggattgaac ccatttttag aggtcagagt tacagacaca     780
   ccgaaacggt cccgcagaga ttttggcctt gactgtgatg agcactcaac ggaatcccga     840
   tgttgtcgct acccgctgac agtggatttc gaagcttttg gatgggactg gattatagca     900
   cctaaaagat acaaagccaa ttactgctcc ggagaatgtg aatttgtgtt tctacagaaa     960
   tacccgcaca ctcacctggt acaccaagca aatcccagag gctcagcagg cccttgctgc    1020
   acacccacca agatgtcccc tataaacatg ctgtatttca atggaaaaga acaaataata    1080
   tatggaaaga taccagccat ggttgtagat cgttgcgggt gctcatga                 1128
<210> 35
   <211> 1128
   <212> DNA
   <213> Anas platyrhynchos
<400> 35
   atgcaaaagc tagcagtcta tgtttatatt tacctgttca tgctgatttc agttgatccg        60
   gtggctcttg atgacggcag tcagcccaca gagaacgctg aaaaagatgg actgtgcaat       120
   gcttgtacgt ggagacagaa tacaaaatct tccagaatag aagccataaa aattcaaatc       180
   ctcagcaaac tgcgcctgga acaagctcct aacattagca gggatgttat taagcaactt       240
   ttacccaaag ctcctccact acaggaactg attgatcaat atgacgtcca gagagacgac       300
   agtagcgatg gctctttgga agacgatgac tatcatgcca caactgaaac gattatcaca       360
   atgcctacag agtctgattt tcttgtacaa atggagggaa aaccaaaatg ttgcttcttt       420
   aagtttagct ctaaaataca atataacaaa gtagtaaagg cacaattgtg gatatacttg       480
   aggcaagtcc aaaaacctac aacagtgttt gtgcagatcc tgagacttat taagcccatg       540
   aaagacggtc caagatatac tggaattcga tctttgaaac ttgacatgaa cccaggcact       600
   ggtatttggc agagtattga tgtgaagaca gtgttgcaaa attggctcaa acagcctgaa       660
   tccaatttag gcatcgaaat aaaagctttt gatgagaatg gacgagatct tgctgtaact       720
   ttcccaggac caggtgaaga tggattgaat ccatttttag aggtcagagt tacagacaca       780
   ccgaaacgat cccgcagaga ttttggcctt gactgcgatg agcactcgac agaatcccga       840
   tgttgtcgct acccactgac cgtggatttt gaagcttttg gatgggactg gattatcgcc       900
   cctaaaagat acaaagccaa ttactgctct gaagaatgcg aatttgtatt tctacagaaa       960
   tacccgcaca ctcatcttgt gcaccaagcg aatcctagag gatcggcagg cccctgctgc      1020
   acgcccacca agatgtcccc cataaatatg ttgtatttca atggaaaaga acaaataata      1080
   tacggaaaga taccagccat ggttgtagat cgttgcgggt gctcatga                  1128
<210> 36
   <211> 1128
   <212> DNA
   <213> Anser anser
<400> 36
   atgcaaaagc tagcagtcta tgtttatatt tacctgttca tgctgatttc agttgatccg        60
   gtggctcttg atgacggtag tcagcccaca gagaatgctg aaaaagatgg actgtgcaat       120
   gcttgtacat ggagacagaa tacaaaatct tccagaatag aagccataaa aattcaaatc       180
   ctcagcaaac tgcgtctgga acaagctcct aacattagca gggatgttat taaacaactt      240
   ttacccaaag ctcctccact acaggaactg attgatcaat atgacgtcca gagagatgac      300
   agtagcgatg gctctttgga agacgatgac tatcatgcca caactgaaac gattatcaca      360
   atgcctacag agtctgattt tcttgtacaa atggagggaa aaccaaaatg ttgcttcttt      420
   aagtttagct ctaaaataca atataacaaa gtagtaaagg cacaattgtg gatttacttg      480
   aggcaagtcc aaaaacctac aacagtgttt gtgcagatcc tgagacttat taagcccatg      540
   aaagacggga caagatatac tggaattcgg tctttgaaac ttgacattga cccaggcgct      600
   ggttttgggc agagtattga tgtgaagaca gtgttgcaaa attggctcaa acagcctgaa      660
   tccaatttag gcatcgaaat aaaagctttt gatgagaatg gacgagatct tgctgtaact      720
   ttcccaggac caggtgaaga tggattgaat ccatttttag aggtcagagt tacagacaca      780
   ccgaaacgat cccgcagaga ttttggcctt gactgcgatg agcactcgac agaatccaga      840
   tgttgtcgct acccactaac tgtggatttt gaagcttttg gatgggactg gattatcgca      900
   cctaaaagat acaaagccaa ttactgcttt ggagaatgtg aatttgtatt tctacagaaa      960
   tatccgcaca ctcatcttgt acaccaagca aatcctagag gctcggcagg cccctgctgc     1020
   acgcccacca agatgtcccc cataaatatg ttgtatttca atggaaaaga acaaataata     1080
   tacggaaaga taccagccat ggttgtagat cgttgcgggt gctcatga                  1128
<210> 37
   <211> 1140
   <212> DNA
   <213> Cervus elaphus
<400> 37
   gattttaaaa ccatgcaaaa actgcaaatc tgtgtttata tttacctatt tatgctgatt      60
   gttgctggcc cagtggatct gaatgagaac agcgagcaga aggaaaatgt ggaaaaagag     120
   gggctgtgta atgcatgttt gtggagacaa aacactaaat ccttaaggct agaagccata     180
   aaaatccaaa tcctcagtaa acttcgcctg gaaacagctc ctaacatcag caaagatgct     240
   ataagacaac ttctgcccaa agctcctcca ctccgggaac tgattgatca gtacgatgtc     300
   cagagagatg acagcagtga cggctccttg gaagatgatg actaccacgc tacgacggaa     360
   acggtcatta ccatgcccac ggagtctgat cttctaacgc aagtggaagg aaaacccaaa     420
   tgttgcttct ttcagtttag ctctaagata caatacaata aagtcgtaaa ggcccaactg     480
   tggatatatc tgagacctgt caagactcct acgacggtgt ttgtgcaaat cctgagactc     540
   atcaaaccca tgaaagacgg tacaaggtat actggaatcc gatctctgaa acttgacatg     600
   aacccaggca ctggtatttg gcagagcatt gacgtgaaga cagtgttgca aaactggctc     660
   aaacaacctg aatccaactt aggcattgag atcaaagctt tagatgagaa tggccatgat     720
   cttgctgtaa ccttcccaga accaggagaa gatggactga atcctttttt agaagtcaag     780
   gtaacagaca caccaaaaag atctaggaga gattttgggc tcgattgtga tgagcactcc     840
   acagaatctc gatgctgtcg ttaccctcta actgtggatt ttgaagctct tggatgggat     900
   tggattattg cacctaaaag atataaggcc aattactgct ctggagaatg tgaatttgtg     960
   tttttgcaaa agtatcctca tacccatctt gtgcaccaag cgaaccccag tggttcagcc    1020
   ggcccctgct gtactcctac aaagatgtct ccaattaata tgctatattt taatgacaaa    1080
   gaacaaataa tacatgggaa gattccagct atggtagtag atcgctgtgg gtgctcatga    1140
<210> 38
   <211> 1128
   <212> DNA
   <213> Antilocapra americana
<400> 38
   atgcaaaaac tgcaaatcta tgtttatatt tacctatcta tgctgattgt tgctggcccc      60
   gtggatctga atgagaacag cgagcagaag gaaaatgggg aaaaagaggg gctgtgtaat     120
   gcatgtttgt ggagacaaaa cactaaatcc tcaagactag aagccataaa aatccaaatc     180
   ctcagtaaac tgcgcctgga aacagctcct aacatcagca aagatgctat aagacaactt     240
   ttgcccaaag ctcccccgct ccgggaactg atcgatcagt acgacgtcca gagagatgac     300
   agcagtgacg gctccttgga agacgatgac taccacgcta cgacggaaac ggtcattacc     360
   atgcccacgg agtctgatct tctaactcaa gtggaaggaa aacccaaatg ttgcttcttt     420
   caatttagct ctaagataca atacaataaa gtagtaaagg cccagctgtg gatatatctg     480
   agacctgtca agactcctac gacagtgttt gtacaaatcc tgagactcat caaacccatg     540
   aaagacggta caaggtatac tggaatccga tctctgaagc ttgacatgaa cccaggcgct     600
   ggtatttggc agagcattga tgtgaagaca gtgttgcaaa actggctcaa acaacctgaa     660
   tccaacttag gcattgaaat caaagcttta gatgagaatg gccatgatct tgctgtaacc     720
   ttcccagaac caggagaaga tggactgaac ccttttttag aagtcaaggt agcagacaca      780
   ccaaaaagat ctaggagaga ttttgggctc gattgtgatg agcactccac agaatctcga     840
   tgctgtcgtt accctctaac tgtggatttt gaagcttttg gatgggattg gatcattgca     900
   cctaaaagat ataaggccaa ttactgctct ggagaatgtg aatttgtgtt tttacaaaag     960
   tatcctcata cccatcttgt gcaccaagca aaccccagag gttcagcagg cccctgctgt    1020
   actcctacaa agatgtctcc aattaatatg ctatatttta atggcaaaga acaaataata    1080
   tacgggaaga ttccagccat ggtagtagat cgctgtgggt gctcatga                 1128
<210> 39
   <211> 1140
   <212> DNA
   <213> Bos taurus
<400> 39
   gattttaaaa ccatgcaaaa actgcaaatc tctgtttata tttacctatt tatgctgatt 60
   gttgctggcc cagtggatct gaatgagaac agcgagcaga aggaaaatgt ggaaaaagag 120
   gggctgtgta atgcatgttt gtggagggaa aacactacat cctcaagact agaagccata 180
   aaaatccaaa tcctcagtaa acttcgcctg gaaacagctc ctaacatcag caaagatgct 240
   atcagacaac ttttgcccaa ggctcctcca ctcctggaac tgattgatca gttcgatgtc 300
   cagagagatg ccagcagtga cggctccttg gaagacgatg actaccacgc caggacggaa 360
   acggtcatta ccatgcccac ggagtctgat cttctaacgc aagtggaagg aaaacccaaa 420
   tgttgcttct ttaaatttag ctctaagata caatacaata aactagtaaa ggcccaactg 480
   tggatatatc tgaggcctgt caagactcct gcgacagtgt ttgtgcaaat cctgagactc 540
   atcaaaccca tgaaagacgg tacaaggtat actggaatcc gatctctgaa acttgacatg 600
   aacccaggca ctggtatttg gcagagcatt gatgtgaaga cagtgttgca gaactggctc 660
   aaacaacctg aatccaactt aggcattgaa atcaaagctt tagatgagaa tggccatgat 720
   cttgctgtaa ccttcccaga accaggagaa gatggactga ctcctttttt agaagtcaag 780
   gtaacagaca caccaaaaag atctaggaga gattttgggc ttgattgtga tgaacactcc 840
   acagaatctc gatgctgtcg ttaccctcta actgtggatt ttgaagcttt tggatgggat 900
   tggattattg cacctaaaag atataaggcc aattactgct ctggagaatg tgaatttgta 960
   tttttgcaaa agtatcctca tacccatctt gtgcaccaag caaaccccag aggttcagcc 1020
   ggcccctgct gtactcctac aaagatgtct ccaattaata tgctatattt taatggcgaa 1080
   ggacaaataa tatacgggaa gattccagcc atggtagtag atcgctgtgg gtgttcatga 1140
<210> 40
   <211> 1128
   <212> DNA
   <213> Ovis aries
<400> 40
   atgcaaaaac tgcaaatctt tgtttatatt tacctattta tgctgcttgt tgctggccca 60
   gtggatctga atgagaacag cgagcagaag gaaaatgtgg aaaaaaaggg gctgtgtaat 120
   gcatgcttgt ggagacaaaa caataaatcc tcaagactag aagccataaa aatccaaatc 180
   ctcagtaagc ttcgcctgga aacagctcct aacatcagca aagatgctat aagacaactt 240
   ttgcccaagg ctcctccact ccgggaactg attgatcagt acgatgtcca gagagatgac 300
   agcagcgacg gctccttgga agacgatgac taccacgtta cgacggaaac ggtcattacc 360
   atgcccacgg agtctgatct tctagcagaa gtgcaagaaa aacccaaatg ttgcttcttt 420
   aaatttagct ctaagataca acacaataaa gtagtaaagg cccaactgtg gatatatctg      480
   agacctgtca agactcctac aacagtgttt gtgcaaatcc tgagactcat caaacccatg      540
   aaagacggta caaggtatac tggaatccga tctctgaaac ttgacatgaa cccaggcact      600
   ggtatttggc agagcattga tgtgaagaca gtgttgcaaa actggctcaa acaacctgaa      660
   tccaacttag gcattgaaat caaagcttta gatgagaatg gtcatgatct tgctgtaacc      720
   ttcccagaac caggagaaga aggactgaat ccttttttag aagtcaaggt aacagacaca      780
   ccaaaaagat ctaggagaga ttttgggctt gattgtgatg agcactccac agaatctcga      840
   tgctgtcgtt accctctaac tgtggatttt gaagcttttg gatgggattg gattattgca      900
   cctaaaagat ataaggccaa ttactgctct ggagaatgtg aatttttatt tttgcaaaag      960
   tatcctcata cccatcttgt gcaccaagca aaccccaaag gttcagccgg cccttgctgt     1020
   actcctacaa agatgtctcc aattaatatg ctatatttta atggcaaaga acaaataata     1080
   tatgggaaga ttccaggcat ggtagtagat cgctgtgggt gctcatga                  1128
<210> 41
   <211> 1255
   <212> DNA
   <213> Capra hircus
<400> 41
   ctggtgtggc aagttgtctc tcagactggg caggcattaa cgtttggctt ggcgttactc 60
   aaaagcaaaa gaaaagtaaa aggaagaagt aagagcaagg aaaaagattg tattgatttt 120
   aaaaccatgc aaaaactgca aatctttgtt tatatttacc tatttatgct gcttgttgct 180
   ggcccagtgg atctgaatga gaacagcgag cagaaggaaa atgtggaaaa aaaggggctg 240
   tgtaatgcat gcttgtggag acaaaacaat aaatcctcaa gactagaagc cataaaaatc 300
   caaatcctca gtaagcttcg cctggaaaca gctcctaaca tcagcaaaga tgctataaga 360
   caacttttgc ccaaggctcc tccactccgg gaactgattg atcagtacga tgtccagaga 420
   gatgacagca gcgacggctc cttggaagac gatgactacc acgttacgac ggaaacggtc 480
   attaccatgc ccacggagtc tgatcttcta gcagaagtgc aagaaaaacc caaatgttgc 540
   ttctttaaat ttagctctaa gatacaacac aataaagtag taaaggccca actgtggata 600
   tatctgagac ctgtcaagac tcctacaaca gtgtttgtgc aaatcctgag actcatcaaa 660
   cccatgaaag acggtacaag gtatactgga atccgatctc tgaaacttga catgaaccca 720
   ggcactggta tttggcagag cattgatgtg aagacagtgt tgcaaaactg gctcaaacaa 780
   cctgaatcca acttaggcat tgaaatcaaa gctttagatg agaatggtca tgatcttgct 840
   gtaaccttcc cagaaccagg agaagaagga ctgaatcctt ttttagaagt caaggtaaca 900
   gacacaccaa aaagatctag gagagatttt gggcttgatt gtgatgagca ctccacagaa 960
   tctcgatgct gtcgttaccc tctaactgtg gattttgaag cctttggatg ggattggatt 1020
   attgcaccta aaagatataa ggccaattac tgctctggag aatgtgaatt tttatttttg 1080
   caaaagtatc ctcataccca tcttgtgcac caagcaaacc ccaaaggttc agccggccct 1140
   tgctgtactc ctacaaagat gtctccaatt aatatgctat attttaatgg caaagaacaa 1200
   ataatatatg ggaagattcc aggcatggta gtagatcgct gtgggtgctc atgag 1255
<210> 42
   <211> 1128
   <212> DNA
   <213> Felis catus
<400> 42
   atgcagaaac tgcaaatcta tgtttatatt tacctgttta tgctgattgt tgctggtcca 60
   gtggatctaa atgagaacag tgagcaaaaa gaaaatgtgg aaaaagaggg gctgtgcaat 120
   gcatgtactt ggagacaaaa cactaaatct tcaagaatag aagccataaa aattcagatc 180
   ctcagtaaac ttcgcctgga aacggctccc aacatcagca aagatgctat aagacaactt 240
   ttgcccaaag ctcctccgct ccgggaactg attgatcagt acgatgtcca gagagatgac 300
   agcagtgatg gctctttgga agacgacgat taccacgcta ccacggaaac gatcattacc 360
   atgcccaccg agtctgatct tctaatgcaa gtggaaggaa aacccaaatg ttgcttcttt 420
   aaatttagct ctaaaataca atataataaa gtcgtaaagg cccaactgtg gatatatctg 480
   agacccgtca agactcctac aacagtgttt gtgcaaatcc tgagactcat caaacccatg 540
   aaagacggta caaggtatac tggaatccga tctctgaaac ttgacatgaa cccaggcact 600
   ggtatttggc agagcattga tgtgaagaca gtgttgcaaa attggctcaa acagcctgaa 660
   tccaacttag gcattgaaat caaagcttta gatgagaatg gtcatgatct tgctgtaacc 720
   ttcccaggac caggagaaga tgggctgaat ccctttttag aagtcaaggt gacagacaca 780
   ccaaaaagat ccagacgaga ttttgggctt gactgtgatg agcactcaac agaatctcgg 840
   tgctgtcgtt accctctcac tgtggatttt gaagcttttg gatgggattg gattattgca 900
   cccaaaagat ataaggccaa ttactgctct ggagagtgtg aatttgtgtt tttacaaaaa 960
   tatcctcata ctcatcttgt acaccaagca aaccccagag gttcagcagg cccctgctgt 1020
   actcccacca agatgtctcc aattaatatg ctatatttta atggcaagga acaaataata 1080
   tatgggaaaa ttccagccat ggtagtagat cgctgtgggt gctcatga 1128
<210> 43
   <211> 1128
   <212> DNA
   <213> Equus caballus
<400> 43
   atgcaaaaac tgcaaatctc tgtttatatt tacctgtttg tgctgattct tgctggtcca 60
   gtggatctaa atgagaacag cgagcaaaaa gaaaatgtgg aaaaagaggg gctgtgcaat      120
   gcatgtactt ggagacaaaa cactaaatct tcaagaatag aagccataaa aattcagatc      180
   ctcagtaaac tgcgcctgga aacagctcct aacatcagca aagatgctat tagacaactt      240
   ttgcccaaag ctcctccact ccgggaactg attgatcagt acgatgtcca gagagatgac      300
   agcagtgatg gctctttgga agatgatgat taccacgcga cgacggaaac aatcattacc      360
   atgcctacag agtctgatct tctaatgcaa gtggaaggaa aacccaaatg ttgcttcttt      420
   aaatttagct ctaaaataca atacaataaa gtagtaaagg cccaactgtg gatatatctg      480
   agacccgtca agactcctac aacagtgttt gtgcaaatcc tgagactcat caaacccatg      540
   aaagacggta caaggtatac tggaatccga tctctgaaac ttgacatgaa cccaggcgct      600
   ggtatttggc agagcattga tgtgaagaca gtgttgcaaa attggctcaa acagcctgaa      660
   tccaacttag gcattgaaat caaagcttta gatgagaatg gtcatgatct tgctgtaacc      720
   ttcccaagac caggagaaga tgggctgaat ccatttttag aagttaaggt aacagacaca      780
   ccaaaacgat ccagaagaga ttttggactt gactgtgatg agcactccac agaatctcga      840
   tgctgtcgtt accctctaac tgtggatttt gaagcttttg gatgggattg gattattgca      900
   cccaaaagat ataaggccaa ttactgctct ggagagtgtg aatttgtatt tttacaaaaa      960
   tatcctcaca ctcatcttgt acaccaagca aaccccagag gttcagcagg cccctgctgt     1020
   actcccacaa agatgtctcc aattaatatg ctatatttta atggcaaaga acaaataata     1080
   tatgggaaaa ttccagccat ggtagtagat cgctgtgggt gctcatga                  1128
<210> 44
   <211> 1128
   <212> DNA
   <213> Sus scrofa
<400> 44
   atgcaaaaac tgcaaatcta tgtttatatt tacctgttta tgctgattgt tgctggtccc      60
   gtggatctga atgagaacag cgagcaaaag gaaaatgtgg aaaaagaggg gctgtgtaat     120
   gcatgtatgt ggagacaaaa cactaaatct tcaagactag aagccataaa aattcaaatc     180
   ctcagtaaac ttcgcctgga aacagctcct aacattagca aagatgctat aagacaactt     240
   ttgcccaaag ctcctccact ccgggaactg attgatcagt acgatgtcca gagagatgac     300
   agcagtgatg gctccttgga agatgatgat tatcacgcta cgacggaaac gatcattacc     360
   atgcctacag agtctgatct tctaatgcaa gtggaaggaa aacccaaatg ctgcttcttt     420
   aaatttagct ctaaaataca atacaataaa gtagtaaagg cccaactgtg gatatatctg     480
   agacccgtca agactcctac aacagtgttt gtgcaaatcc tgagactcat caaacccatg     540
   aaagacggta caaggtatac tggaatccga tctctgaaac ttgacatgaa cccaggcact     600
   ggtatttggc agagcattga tgtgaagaca gtgttgcaaa attggctcaa acaacctgaa     660
   tccaacttag gcattgaaat caaagcttta gatgagaatg gtcatgatct tgctgtaacc     720
   ttcccaggac caggagaaga tgggctgaat ccctttttag aagtcaaggt aacagacaca     780
   ccaaaaagat ccaggagaga ttttggactc gactgtgatg agcactcaac agaatctcga     840
   tgctgtcgtt accctctaac tgtggatttt gaagcttttg gatgggactg gattattgca     900
   cccaaaagat ataaggccag ttactgctct ggagagtgtg aatttgtatt tttacaaaaa     960
   taccctcaca ctcatcttgt gcaccaagca aaccccagag gttcagcagg cccctgctgt    1020
   actcccacaa agatgtctcc aatcaatatg ctatatttta atggcaaaga acaaataata    1080
   tatgggaaaa ttccagccat ggtagtagat cgctgtgggt gctcatga                 1128
<210> 45
   <211> 18
   <212> DNA
   <213> Equus Caballus
<400> 45
   acaccgcccg tcaccctc 18
<210> 46
   <211> 20
   <212> DNA
   <213> Equus Caballus
<400> 46
   gtaccgtaag ggaacgatga 20
<210> 47
   <211> 18
   <212> DNA
   <213> Felis Catus
<400> 47
   acaccgcccg tcaccctc 18
<210> 48
   <211> 20
   <212> DNA
   <213> Felis Catus
<400> 48
   gtaccgtaag ggaacgatga 20
<210> 49
   <211> 18
   <212> DNA
   <213> BT1
<400> 49
   acaccgcccg tcaccctc 18
<210> 50
   <211> 20
   <212> DNA
   <213> BT1
<400> 50
   gtaccgcaag ggaacgatga 20
<210> 51
   <211> 18
   <212> DNA
   <213> BT2
<400> 51
   acaccgcccg tcaccctc 18
<210> 52
   <211> 20
   <212> DNA
   <213> BT2
<400> 52
   gtaccgcaag ggaacgatga 20
<210> 53
   <211> 18
   <212> DNA
   <213> Capra Hircus
<400> 53
   acaccgcccg tcaccctc 18
<210> 54
   <211> 20
   <212> DNA
   <213> Capra Hircus
<400> 54
   gtaccgtaag ggaatgatga 20
<210> 55
   <211> 18
   <212> DNA
   <213> Canis Lupisfamiliaris
<400> 55
   acaccgcccg tcaccctc 18
<210> 56
   <211> 20
   <212> DNA
   <213> Canis Lupusfamiliaris
<400> 56
   gtaccgtaag ggaatgatga 20
<210> 57
   <211> 18
   <212> DNA
   <213> SS-Pietrain
<400> 57
   acaccgcccg tcaccctc 18
<210> 58
   <211> 20
   <212> DNA
   <213> SS-Pietrain
<400> 58
   gtaccgtaag ggaaagatga 20
<210> 59
   <211> 18
   <212> DNA
   <213> SS-LW
<400> 59
   acaccgcccg tcaccctc 18
<210> 60
   <211> 20
   <212> DNA
   <213> SS-LW
<400> 60
   gtaccgtaag ggaaagatga 20
<210> 61
   <211> 18
   <212> DNA
   <213> Gallus Gallus 1
<400> 61
   ataccgcccg tcaccctc 18
<210> 62
   <211> 20
   <212> DNA
   <213> Gallus Gallus 1
<400> 62
   gtaccgcaag ggaaagatga 20
<210> 63
   <211> 18
   <212> DNA
   <213> Gallus Gallus 2
<400> 63
   ataccgcccg tcaccctc 18
<210> 64
   <211> 20
   <212> DNA
   <213> Gallus Gallus 2
<400> 64
   gtaccgcaag ggaaagatga 20
<210> 65
   <211> 18
   <212> DNA
   <213> Meleagris Gallopavo
<400> 65
   ataccgcccg tcaccctc 18
<210> 66
   <211> 20
   <212> DNA
   <213> Meleagris Gallopavo
<400> 66
   gtaccgtaag ggaaagatga 20
<210> 67
   <211> 18
   <212> DNA
   <213> Anser Anser
<400> 67
   ataccgcccg tcaccctc 18
<210> 68
   <211> 20
   <212> DNA
   <213> Anser Anser
<400> 68
   gtaccgtaag ggaaagatga 20
<210> 69
   <211> 18
   <212> DNA
   <213> Anas Platyrhynchos
<400> 69
   ataccgcccg tcaccctc 18
<210> 70
   <211> 20
   <212> DNA
   <213> Anas Platyrhynchos
<400> 70
   gtaccgtaag ggaaagatga 20
<210> 71
   <211> 18
   <212> DNA
   <213> artificial
<220>
   <223> primer
<400> 71
   ayaccgcccg tcaccctc 18
<210> 72
   <211> 18
   <212> DNA
   <213> artificial
<220>
   <223> primer
<400> 72
   twggcttttc acctctac 18
<210> 73
   <211> 18
   <212> DNA
   <213> artificial
<220>
   <223> primer
<400> 73
   twggcatgtc acctctac 18
<210> 74
   <211> 57
   <212> DNA
   <213> artificial
<220>
   <223> primer
<400> 74
   ccatctcatc cctgcgtgtc tccgactcag agactgcaca yaccgcccgt caccctc 57
<210> 75
   <211> 57
   <212> DNA
   <213> artificial
<220>
   <223> primer
<400> 75
   ccatctcatc cctgcgtgtc tccgactcag agcgcgcgca yaccgcccgt caccctc 57
<210> 76
   <211> 57
   <212> DNA
   <213> artificial
<220>
   <223> primer
<400> 76
   ccatctcatc cctgcgtgtc tccgactcag agctctatca yaccgcccgt caccctc 57
<210> 77
   <211> 48
   <212> DNA
   <213> artificial
<220>
   <223> primer
<400> 77
   cctatcccct gtgtgccttg gcagtctcag twggcttttc acctctac 48
<210> 78
   <211> 48
   <212> DNA
   <213> artificial
<220>
   <223> primer
<400> 78
   cctatcccct gtgtgccttg gcagtctcag twggcatgtc acctctac 48
<210> 79
   <211> 47
   <212> DNA
   <213> artificial
<220>
   <223> primer
<400> 79
   cctatcccct gtgtgccttg gcagtctcag twggcwtktc acctcta 47
<210> 80
   <211> 1140
   <212> DNA
   <213> cervus elaphus
<400> 80
   gattttaaaa ccatgcaaaa actgcaaatc tgtgtttata tttacctatt tatgctgatt 60
   gttgctggcc cagtggatct gaatgagaac agcgagcaga aggaaaatgt ggaaaaagag 120
   gggctgtgta atgcatgttt gtggagacaa aacactaaat ccttaaggct agaagccata 180
   aaaatccaaa tcctcagtaa acttcgcctg gaaacagctc ctaacatcag caaagatgct 240
   ataagacaac ttctgcccaa agctcctcca ctccgggaac tgattgatca gtacgatgtc 300
   cagagagatg acagcagtga cggctccttg gaagatgatg actaccacgc tacgacggaa 360
   acggtcatta ccatgcccac ggagtctgat cttctaacgc aagtggaagg aaaacccaaa 420
   tgttgcttct ttcagtttag ctctaagata caatacaata aagtcgtaaa ggcccaactg 480
   tggatatatc tgagacctgt caagactcct acgacggtgt ttgtgcaaat cctgagactc 540
   atcaaaccca tgaaagacgg tacaaggtat actggaatcc gatctctgaa acttgacatg 600
   aacccaggca ctggtatttg gcagagcatt gacgtgaaga cagtgttgca aaactggctc 660
   aaacaacctg aatccaactt aggcattgag atcaaagctt tagatgagaa tggccatgat 720
   cttgctgtaa ccttcccaga accaggagaa gatggactga atcctttttt agaagtcaag 780
   gtaacagaca caccaaaaag atctaggaga gattttgggc tcgattgtga tgagcactcc 840
   acagaatctc gatgctgtcg ttaccctcta actgtggatt ttgaagctct tggatgggat 900
   tggattattg cacctaaaag atataaggcc aattactgct ctggagaatg tgaatttgtg 960
   tttttgcaaa agtatcctca tacccatctt gtgcaccaag cgaaccccag tggttcagcc 1020
   ggcccctgct gtactcctac aaagatgtct ccaattaata tgctatattt taatgacaaa 1080
   gaacaaataa tacatgggaa gattccagct atggtagtag atcgctgtgg gtgctcatga 1140
<210> 81
   <211> 1128
   <212> DNA
   <213> Antilocapra americana
<400> 81
   atgcaaaaac tgcaaatcta tgtttatatt tacctatcta tgctgattgt tgctggcccc      60
   gtggatctga atgagaacag cgagcagaag gaaaatgggg aaaaagaggg gctgtgtaat     120
   gcatgtttgt ggagacaaaa cactaaatcc tcaagactag aagccataaa aatccaaatc     180
   ctcagtaaac tgcgcctgga aacagctcct aacatcagca aagatgctat aagacaactt     240
   ttgcccaaag ctcccccgct ccgggaactg atcgatcagt acgacgtcca gagagatgac     300
   agcagtgacg gctccttgga agacgatgac taccacgcta cgacggaaac ggtcattacc     360
   atgcccacgg agtctgatct tctaactcaa gtggaaggaa aacccaaatg ttgcttcttt     420
   caatttagct ctaagataca atacaataaa gtagtaaagg cccagctgtg gatatatctg     480
   agacctgtca agactcctac gacagtgttt gtacaaatcc tgagactcat caaacccatg     540
   aaagacggta caaggtatac tggaatccga tctctgaagc ttgacatgaa cccaggcgct     600
   ggtatttggc agagcattga tgtgaagaca gtgttgcaaa actggctcaa acaacctgaa     660
   tccaacttag gcattgaaat caaagcttta gatgagaatg gccatgatct tgctgtaacc     720
   ttcccagaac caggagaaga tggactgaac ccttttttag aagtcaaggt agcagacaca     780
   ccaaaaagat ctaggagaga ttttgggctc gattgtgatg agcactccac agaatctcga     840
   tgctgtcgtt accctctaac tgtggatttt gaagcttttg gatgggattg gatcattgca     900
   cctaaaagat ataaggccaa ttactgctct ggagaatgtg aatttgtgtt tttacaaaag     960
   tatcctcata cccatcttgt gcaccaagca aaccccagag gttcagcagg cccctgctgt    1020
   actcctacaa agatgtctcc aattaatatg ctatatttta atggcaaaga acaaataata    1080
   tacgggaaga ttccagccat ggtagtagat cgctgtgggt gctcatga                 1128
<210> 82
   <211> 1128
   <212> DNA
   <213> coturnix chinensis
<400> 82
   atgcaaaagc tagcagtcta tgtttatatt tacctgttcg tgcagatatc tgttgatccg        60
   gtggctctcg atggcagtag tcagcccaca gagaacactg aaaaagacgg actgtgcaat       120
   gcttgtacgt ggagacagaa cacaaaatcc tccagaatag aagccataaa aattcaaatc       180
   ctcagcaaac tgcgcctgga acaagcacct aacattagca gggacgttat taaacaactt       240
   ctacccaaag ctcctccact gcaggaactg attgatcagt acgacgtcca gagagatgac       300
   agtagcgatg gctctttgga agatgatgac tatcatgcca caaccgaaac gattatcaca       360
   atgcctacgg agtctgattt tcttgtacaa atggagggaa aaccaaaatg ttgcttcttt       420
   aagtttagct ctaagataca atataacaaa gtagtaaagg cacaattatg gatatacttg       480
   aggcaagtcc aaaaacctac aacggtgttt gtgcagatcc tgagactcat taaacccatg       540
   aaagacggta caagatatac tggaattcga tctttgaaac ttgacatgaa cccaggcaat       600
   ggtatctggc agagtattga tgtgaagaca gtcttgcaaa attggctcaa acagcctgaa       660
   tccaacttag gcatcgaaat aaaagctttt gatgagaatg gacgagatct tgctgtaaca       720
   ttcccaggac caggtgaaga cggatcgaac ccatttttag aggtcagagt tacagataca       780
   ccaaaacggt cccgcagaga ttttggcctt gactgtgatg agcactcaac agaatctcga       840
   tgttgtcgct accccttgac ggtggatttt gaagcttttg gatgggactg gattatagcg       900
   cctaaaagat acaaagcaaa ttactgctct ggagaatgcg aatttgtatt tctacagaaa       960
   taccctcaca ctcacctggt gcaccaagca aatccaagag gttcagcagg cccttgctgc      1020
   acacccacca agatgtcccc tataaatatg ctgtatttca atggaaaaga acaaataata      1080
   tatggaaaga taccagctat ggttgtagat cgttgcgggt gctcatga                   1128
<210> 83
   <211> 1014
   <212> DNA
   <213> xenopus tropicalis
<400> 83
   gacaaagata cattgtgcag tgcttgtatc tggagacaga acagtaaatc tacaaggctt        60
   gaagctataa aaacccagat ccttagcaaa cttcgactgg aacaggcacc taatattagc      120
   aaggatgcta taaaacacct tttacccaaa gcaccacctt tacaagattt aatagacaaa      180
   tatgatgttc aaaaagatga aagcagtgct ggtcatttgg aagaggatga ttatcatgtc      240
   tctgctgaaa ctgttattat aatgcctacc gagtttggaa tttccattga tatgaaagaa      300
   aaacctattt gttgcttttt taaattcagt tctaaagttc agttgacaaa aatatcgaaa      360
   gcacagctct ggatacattt aaaacctgtt caaaaaccta cgacagttgt tgtgcagatc      420
   tcgagactca ttaagccttt gaaagatggt acaagataca ctggaatccg ctcactaaaa      480
   cttgaaatga atccaggttc tggaacttgg cagagcatag atgtaaaaac tgtactgcaa      540
   aattggctga ggcaacccga atccaatcta ggcattgaaa taagagcatt tgatggaaat      600
   gggcaggatc ttgcagtaac cagtaatgaa gatggtctga gtccatttat ggaggtcaag      660
   attgtagaca caccaaaacg atttagaaga gactcagggc ttgactgtga tgagcattca      720
   actgaaacta tgtgctgccg atacccatta acagttgatt ttgaggcatt tggctgggac      780
   tgggtggttg ctccaaagag atataatgct aactactgct caggagaatg tggaattgaa      840
   tatctgcaga aatacccaca tggccatgtt gttaatcaag ctaaccccaa aggtcctaca      900
   ggtccatgtt gttccccaac caaaatgtcc tccctaaata tgttatattt taatgatgat      960
   gcagaagtta tccagggaaa gattccagcc atggtaatag atcgctgtgg gtgc           1014
<210> 84
   <211> 1128
   <212> DNA
   <213> Felis catus
<400> 84
   atgcagaaac tgcaaatcta tgtttatatt tacctgttta tgctgattgt tgctggtcca 60
   gtggatctaa atgagaacag tgagcaaaaa gaaaatgtgg aaaaagaggg gctgtgcaat 120
   gcatgtactt ggagacaaaa cactaaatct tcaagaatag aagccataaa aattcagatc 180
   ctcagtaaac ttcgcctgga aacggctccc aacatcagca aagatgctat aagacaactt 240
   ttgcccaaag ctcctccgct ccgggaactg attgatcagt acgatgtcca gagagatgac 300
   agcagtgatg gctctttgga agacgacgat taccacgcta ccacggaaac gatcattacc 360
   atgcccaccg agtctgatct tctaatgcaa gtggaaggaa aacccaaatg ttgcttcttt 420
   aaatttagct ctaaaataca atataataaa gtcgtaaagg cccaactgtg gatatatctg 480
   agacccgtca agactcctac aacagtgttt gtgcaaatcc tgagactcat caaacccatg 540
   aaagacggta caaggtatac tggaatccga tctctgaaac ttgacatgaa cccaggcact 600
   ggtatttggc agagcattga tgtgaagaca gtgttgcaaa attggctcaa acagcctgaa 660
   tccaacttag gcattgaaat caaagcttta gatgagaatg gtcatgatct tgctgtaacc 720
   ttcccaggac caggagaaga tgggctgaat ccctttttag aagtcaaggt gacagacaca 780
   ccaaaaagat ccagacgaga ttttgggctt gactgtgatg agcactcaac agaatctcgg 840
   tgctgtcgtt accctctcac tgtggatttt gaagcttttg gatgggattg gattattgca 900
   cccaaaagat ataaggccaa ttactgctct ggagagtgtg aatttgtgtt tttacaaaaa 960
   tatcctcata ctcatcttgt acaccaagca aaccccagag gttcagcagg cccctgctgt 1020
   actcccacca agatgtctcc aattaatatg ctatatttta atggcaagga acaaataata 1080
   tatgggaaaa ttccagccat ggtagtagat cgctgtgggt gctcatga 1128
<210> 85
   <211> 1128
   <212> **DNA**
   <213> Sus scrofa
<400> 85
   atgcaaaaac tgcaaatcta tgtttatatt tacctgttta tgctgattgt tgctggtccc        60
   gtggatctga atgagaacag cgagcaaaag gaaaatgtgg aaaaagaggg gctgtgtaat       120
   gcatgtatgt ggagacaaaa cactaaatct tcaagactag aagccataaa aattcaaatc       180
   ctcagtaaac ttcgcctgga aacagctcct aacattagca aagatgctat aagacaactt       240
   ttgcccaaag ctcctccact ccgggaactg attgatcagt acgatgtcca gagagatgac       300
   agcagtgatg gctccttgga agatgatgat tatcacgcta cgacggaaac gatcattacc       360
   atgcctacag agtctgatct tctaatgcaa gtggaaggaa aacccaaatg ctgcttcttt       420
   aaatttagct ctaaaataca atacaataaa gtagtaaagg cccaactgtg gatatatctg       480
   agacccgtca agactcctac aacagtgttt gtgcaaatcc tgagactcat caaacccatg       540
   aaagacggta caaggtatac tggaatccga tctctgaaac ttgacatgaa cccaggcact       600
   ggtatttggc agagcattga tgtgaagaca gtgttgcaaa attggctcaa acaacctgaa       660
   tccaacttag gcattgaaat caaagcttta gatgagaatg gtcatgatct tgctgtaacc       720
   ttcccaggac caggagaaga tgggctgaat ccctttttag aagtcaaggt aacagacaca       780
   ccaaaaagat ccaggagaga ttttggactc gactgtgatg agcactcaac agaatctcga       840
   tgctgtcgtt accctctaac tgtggatttt gaagcttttg gatgggactg gattattgca       900
   cccaaaagat ataaggccag ttactgctct ggagagtgtg aatttgtatt tttacaaaaa       960
   taccctcaca ctcatcttgt gcaccaagca aaccccagag gttcagcagg cccctgctgt      1020
   actcccacaa agatgtctcc aatcaatatg ctatatttta atggcaaaga acaaataata      1080
   tatgggaaaa ttccagccat ggtagtagat cgctgtgggt gctcatga                   1128
<210> 86
   <211> 1140
   <212> DNA
   <213> Bos taurus
<400> 86
   gattttaaaa ccatgcaaaa actgcaaatc tctgtttata tttacctatt tatgctgatt 60
   gttgctggcc cagtggatct gaatgagaac agcgagcaga aggaaaatgt ggaaaaagag 120
   gggctgtgta atgcatgttt gtggagggaa aacactacat cctcaagact agaagccata 180
   aaaatccaaa tcctcagtaa acttcgcctg gaaacagctc ctaacatcag caaagatgct 240
   atcagacaac ttttgcccaa ggctcctcca ctcctggaac tgattgatca gttcgatgtc 300
   cagagagatg ccagcagtga cggctccttg gaagacgatg actaccacgc caggacggaa 360
   acggtcatta ccatgcccac ggagtctgat cttctaacgc aagtggaagg aaaacccaaa 420
   tgttgcttct ttaaatttag ctctaagata caatacaata aactagtaaa ggcccaactg 480
   tggatatatc tgaggcctgt caagactcct gcgacagtgt ttgtgcaaat cctgagactc 540
   atcaaaccca tgaaagacgg tacaaggtat actggaatcc gatctctgaa acttgacatg 600
   aacccaggca ctggtatttg gcagagcatt gatgtgaaga cagtgttgca gaactggctc 660
   aaacaacctg aatccaactt aggcattgaa atcaaagctt tagatgagaa tggccatgat 720
   cttgctgtaa ccttcccaga accaggagaa gatggactga ctcctttttt agaagtcaag 780
   gtaacagaca caccaaaaag atctaggaga gattttgggc ttgattgtga tgaacactcc 840
   acagaatctc gatgctgtcg ttaccctcta actgtggatt ttgaagcttt tggatgggat 900
   tggattattg cacctaaaag atataaggcc aattactgct ctggagaatg tgaatttgta 960
   tttttgcaaa agtatcctca tacccatctt gtgcaccaag caaaccccag aggttcagcc 1020
   ggcccctgct gtactcctac aaagatgtct ccaattaata tgctatattt taatggcgaa 1080
   ggacaaataa tatacgggaa gattccagcc atggtagtag atcgctgtgg gtgttcatga 1140
<210> 87
   <211> 1128
   <212> **DNA**
   <213> Gallus gallus
<400> 87
   atgcaaaagc tagcagtcta tgtttatatt tacctgttca tgcagatcgc ggttgatcca 60
   gtggctctgg atggcagtag tcagcccaca gagaacgctg aaaaagacgg actgtgcaat 120
   gcttgtacgt ggagacagaa tacaaaatcc tccagaatag aagccataaa aattcaaatc 180
   ctcagcaaac tgcgcctgga acaagcacct aacattagca gggacgttat taagcagctt 240
   ttacccaaag ctcctccact gcaggaactg attgatcagt atgatgtcca gagggacgac 300
   agtagcgatg gctctttgga agacgatgac tatcatgcca caaccgagac gattatcaca 360
   atgcctacgg agtctgattt tcttgtacaa atggagggaa aaccaaaatg ttgcttcttt 420
   aagtttagct ctaaaataca atataacaaa gtagtaaagg cacaattatg gatatacttg 480
   aggcaagtcc aaaaacctac aacggtgttt gtgcagatcc tgagactcat taagcccatg      540
   aaagacggta caagatatac tggaattcga tctttgaaac ttgacatgaa cccaggcact      600
   ggtatctggc agagtattga tgtgaagaca gtgctgcaaa attggctcaa acagcctgaa      660
   tccaatttag gcatcgaaat aaaagctttt gatgagactg gacgagatct tgctgtcaca      720
   ttcccaggac cgggtgaaga tggattgaac ccatttttag aggtcagagt tacagacaca      780
   ccgaaacggt cccgcagaga ttttggcctt gactgtgatg agcactcaac ggaatcccga      840
   tgttgtcgct acccgctgac agtggatttc gaagcttttg gatgggactg gattatagca      900
   cctaaaagat acaaagccaa ttactgctcc ggagaatgtg aatttgtgtt tctacagaaa      960
   tacccgcaca ctcacctggt acaccaagca aatcccagag gctcagcagg cccttgctgc     1020
   acacccacca agatgtcccc tataaacatg ctgtatttca atggaaaaga acaaataata     1080
   tatggaaaga taccagccat ggttgtagat cgttgcgggt gctcatga                  1128
<210> 88
   <211> 1128
   <212> DNA
   <213> Equus caballus
<400> 88
   atgcaaaaac tgcaaatctc tgtttatatt tacctgtttg tgctgattct tgctggtcca 60
   gtggatctaa atgagaacag cgagcaaaaa gaaaatgtgg aaaaagaggg gctgtgcaat 120
   gcatgtactt ggagacaaaa cactaaatct tcaagaatag aagccataaa aattcagatc 180
   ctcagtaaac tgcgcctgga aacagctcct aacatcagca aagatgctat tagacaactt 240
   ttgcccaaag ctcctccact ccgggaactg attgatcagt acgatgtcca gagagatgac 300
   agcagtgatg gctctttgga agatgatgat taccacgcga cgacggaaac aatcattacc 360
   atgcctacag agtctgatct tctaatgcaa gtggaaggaa aacccaaatg ttgcttcttt 420
   aaatttagct ctaaaataca atacaataaa gtagtaaagg cccaactgtg gatatatctg 480
   agacccgtca agactcctac aacagtgttt gtgcaaatcc tgagactcat caaacccatg 540
   aaagacggta caaggtatac tggaatccga tctctgaaac ttgacatgaa cccaggcgct 600
   ggtatttggc agagcattga tgtgaagaca gtgttgcaaa attggctcaa acagcctgaa 660
   tccaacttag gcattgaaat caaagcttta gatgagaatg gtcatgatct tgctgtaacc 720
   ttcccaagac caggagaaga tgggctgaat ccatttttag aagttaaggt aacagacaca 780
   ccaaaacgat ccagaagaga ttttggactt gactgtgatg agcactccac agaatctcga 840
   tgctgtcgtt accctctaac tgtggatttt gaagcttttg gatgggattg gattattgca 900
   cccaaaagat ataaggccaa ttactgctct ggagagtgtg aatttgtatt tttacaaaaa 960
   tatcctcaca ctcatcttgt acaccaagca aaccccagag gttcagcagg cccctgctgt 1020
   actcccacaa agatgtctcc aattaatatg ctatatttta atggcaaaga acaaataata 1080
   tatgggaaaa ttccagccat ggtagtagat cgctgtgggt gctcatga 1128
<210> 89
   <211> 1128
   <212> DNA
   <213> Ovis aries
<400> 89
   atgcaaaaac tgcaaatctt tgtttatatt tacctattta tgctgcttgt tgctggccca      60
   gtggatctga atgagaacag cgagcagaag gaaaatgtgg aaaaaaaggg gctgtgtaat     120
   gcatgcttgt ggagacaaaa caataaatcc tcaagactag aagccataaa aatccaaatc     180
   ctcagtaagc ttcgcctgga aacagctcct aacatcagca aagatgctat aagacaactt     240
   ttgcccaagg ctcctccact ccgggaactg attgatcagt acgatgtcca gagagatgac     300
   agcagcgacg gctccttgga agacgatgac taccacgtta cgacggaaac ggtcattacc     360
   atgcccacgg agtctgatct tctagcagaa gtgcaagaaa aacccaaatg ttgcttcttt     420
   aaatttagct ctaagataca acacaataaa gtagtaaagg cccaactgtg gatatatctg     480
   agacctgtca agactcctac aacagtgttt gtgcaaatcc tgagactcat caaacccatg     540
   aaagacggta caaggtatac tggaatccga tctctgaaac ttgacatgaa cccaggcact     600
   ggtatttggc agagcattga tgtgaagaca gtgttgcaaa actggctcaa acaacctgaa     660
   tccaacttag gcattgaaat caaagcttta gatgagaatg gtcatgatct tgctgtaacc     720
   ttcccagaac caggagaaga aggactgaat ccttttttag aagtcaaggt aacagacaca     780
   ccaaaaagat ctaggagaga ttttgggctt gattgtgatg agcactccac agaatctcga     840
   tgctgtcgtt accctctaac tgtggatttt gaagcttttg gatgggattg gattattgca     900
   cctaaaagat ataaggccaa ttactgctct ggagaatgtg aatttttatt tttgcaaaag     960
   tatcctcata cccatcttgt gcaccaagca aaccccaaag gttcagccgg cccttgctgt    1020
   actcctacaa agatgtctcc aattaatatg ctatatttta atggcaaaga acaaataata    1080
   tatgggaaga ttccaggcat ggtagtagat cgctgtgggt gctcatga                1128
<210> 90
   <211> 1255
   <212> DNA
   <213> Capra hircus
<400> 90
   ctggtgtggc aagttgtctc tcagactggg caggcattaa cgtttggctt ggcgttactc      60
   aaaagcaaaa gaaaagtaaa aggaagaagt aagagcaagg aaaaagattg tattgatttt     120
   aaaaccatgc aaaaactgca aatctttgtt tatatttacc tatttatgct gcttgttgct     180
   ggcccagtgg atctgaatga gaacagcgag cagaaggaaa atgtggaaaa aaaggggctg      240
   tgtaatgcat gcttgtggag acaaaacaat aaatcctcaa gactagaagc cataaaaatc      300
   caaatcctca gtaagcttcg cctggaaaca gctcctaaca tcagcaaaga tgctataaga      360
   caacttttgc ccaaggctcc tccactccgg gaactgattg atcagtacga tgtccagaga      420
   gatgacagca gcgacggctc cttggaagac gatgactacc acgttacgac ggaaacggtc      480
   attaccatgc ccacggagtc tgatcttcta gcagaagtgc aagaaaaacc caaatgttgc      540
   ttctttaaat ttagctctaa gatacaacac aataaagtag taaaggccca actgtggata      600
   tatctgagac ctgtcaagac tcctacaaca gtgtttgtgc aaatcctgag actcatcaaa      660
   cccatgaaag acggtacaag gtatactgga atccgatctc tgaaacttga catgaaccca      720
   ggcactggta tttggcagag cattgatgtg aagacagtgt tgcaaaactg gctcaaacaa      780
   cctgaatcca acttaggcat tgaaatcaaa gctttagatg agaatggtca tgatcttgct      840
   gtaaccttcc cagaaccagg agaagaagga ctgaatcctt ttttagaagt caaggtaaca      900
   gacacaccaa aaagatctag gagagatttt gggcttgatt gtgatgagca ctccacagaa      960
   tctcgatgct gtcgttaccc tctaactgtg gattttgaag cctttggatg ggattggatt     1020
   attgcaccta aaagatataa ggccaattac tgctctggag aatgtgaatt tttatttttg     1080
   caaaagtatc ctcataccca tcttgtgcac caagcaaacc ccaaaggttc agccggccct     1140
   tgctgtactc ctacaaagat gtctccaatt aatatgctat attttaatgg caaagaacaa     1200
   ataatatatg ggaagattcc aggcatggta gtagatcgct gtgggtgctc atgag          1255
<210> 91
   <211> 1128
   <212> DNA
   <213> Anas platyrhynchos
<400> 91
   atgcaaaagc tagcagtcta tgtttatatt tacctgttca tgctgatttc agttgatccg      60
   gtggctcttg atgacggcag tcagcccaca gagaacgctg aaaaagatgg actgtgcaat     120
   gcttgtacgt ggagacagaa tacaaaatct tccagaatag aagccataaa aattcaaatc     180
   ctcagcaaac tgcgcctgga acaagctcct aacattagca gggatgttat taagcaactt     240
   ttacccaaag ctcctccact acaggaactg attgatcaat atgacgtcca gagagacgac     300
   agtagcgatg gctctttgga agacgatgac tatcatgcca caactgaaac gattatcaca     360
   atgcctacag agtctgattt tcttgtacaa atggagggaa aaccaaaatg ttgcttcttt     420
   aagtttagct ctaaaataca atataacaaa gtagtaaagg cacaattgtg gatatacttg     480
   aggcaagtcc aaaaacctac aacagtgttt gtgcagatcc tgagacttat taagcccatg     540
   aaagacggtc caagatatac tggaattcga tctttgaaac ttgacatgaa cccaggcact     600
   ggtatttggc agagtattga tgtgaagaca gtgttgcaaa attggctcaa acagcctgaa     660
   tccaatttag gcatcgaaat aaaagctttt gatgagaatg gacgagatct tgctgtaact     720
   ttcccaggac caggtgaaga tggattgaat ccatttttag aggtcagagt tacagacaca     780
   ccgaaacgat cccgcagaga ttttggcctt gactgcgatg agcactcgac agaatcccga     840
   tgttgtcgct acccactgac cgtggatttt gaagcttttg gatgggactg gattatcgcc     900
   cctaaaagat acaaagccaa ttactgctct gaagaatgcg aatttgtatt tctacagaaa     960
   tacccgcaca ctcatcttgt gcaccaagcg aatcctagag gatcggcagg cccctgctgc    1020
   acgcccacca agatgtcccc cataaatatg ttgtatttca atggaaaaga acaaataata    1080
   tacggaaaga taccagccat ggttgtagat cgttgcgggt gctcatga                 1128
<210> 92
   <211> 1128
   <212> DNA
   <213> Anser anser
<400> 92
   atgcaaaagc tagcagtcta tgtttatatt tacctgttca tgctgatttc agttgatccg      60
   gtggctcttg atgacggtag tcagcccaca gagaatgctg aaaaagatgg actgtgcaat     120
   gcttgtacat ggagacagaa tacaaaatct tccagaatag aagccataaa aattcaaatc     180
   ctcagcaaac tgcgtctgga acaagctcct aacattagca gggatgttat taaacaactt     240
   ttacccaaag ctcctccact acaggaactg attgatcaat atgacgtcca gagagatgac     300
   agtagcgatg gctctttgga agacgatgac tatcatgcca caactgaaac gattatcaca     360
   atgcctacag agtctgattt tcttgtacaa atggagggaa aaccaaaatg ttgcttcttt     420
   aagtttagct ctaaaataca atataacaaa gtagtaaagg cacaattgtg gatttacttg     480
   aggcaagtcc aaaaacctac aacagtgttt gtgcagatcc tgagacttat taagcccatg     540
   aaagacggga caagatatac tggaattcgg tctttgaaac ttgacattga cccaggcgct     600
   ggttttgggc agagtattga tgtgaagaca gtgttgcaaa attggctcaa acagcctgaa     660
   tccaatttag gcatcgaaat aaaagctttt gatgagaatg gacgagatct tgctgtaact     720
   ttcccaggac caggtgaaga tggattgaat ccatttttag aggtcagagt tacagacaca     780
   ccgaaacgat cccgcagaga ttttggcctt gactgcgatg agcactcgac agaatccaga     840
   tgttgtcgct acccactaac tgtggatttt gaagcttttg gatgggactg gattatcgca     900
   cctaaaagat acaaagccaa ttactgcttt ggagaatgtg aatttgtatt tctacagaaa     960
   tatccgcaca ctcatcttgt acaccaagca aatcctagag gctcggcagg cccctgctgc    1020
   acgcccacca agatgtcccc cataaatatg ttgtatttca atggaaaaga acaaataata    1080
   tacggaaaga taccagccat ggttgtagat cgttgcgggt gctcatga                1128
<210> 93
   <211> 288
   <212> DNA
   <213> Cairina moschata
<400> 93
   tgcgatgagc actcgacaga atcccgatgt tgtcgctacc cactgaccgt ggattttgaa      60
   gcctttggat gggactggat tatcgcccct aaaagataca aagccaatta ctgctctgga     120
   gaatgcgaat ttgtatttct acagaaatac ccgcacactc atcttgtaca ccaagcaaat     180
   cctagaggct cggcaggccc ctgctgcacg cccaccaaga tgtcccccat aaatatgttg     240
   tatttcaatg gaaaagaaca aataatatac ggaaagatac cagccatg                  288
<210> 94
   <211> 634
   <212> DNA
   <213> Capra hircus
<400> 94
   ggatacggcg taaacgtgtt aaagcactac atcaaataga gttaaattct aattaaactg      60
   taaaaagcca taattacaac aaaaataaat gacgaaagta accctactgc agctgataca     120
   ctatagctaa gacccaaact gggattagat accccactat gcttagccct aaacacaaat     180
   aattacagaa acaaaattat tcgccagagt actaccggca acagcccgaa actcaaagga     240
   cttggcggtg ctttataccc ttctagagga gcctgttcta taatcgataa accccgataa     300
   acctcaccaa tccttgctaa tacagtctat ataccgccat cttcagcaaa ccctaaaaag     360
   gaacaaaagt aagctcaatc acaacacata aagacgttag gtcaaggtgt aacccatgga     420
   atgggaagaa atgggctaca ttttctacct taagaaaatt aatacgaaag ccattatgaa     480
   attaatgacc aaaggaggat ttagtagtaa actaagaata gagtgcttag ttgaattagg     540
   ccatgaagca cgcacacacc gcccgtcacc ctcctcaagt aaatacaatg cactcaagcc     600
   tattaacacg catcaactac atgagaggag ataa                                 634
<210> 95
   <211> 954
   <212> DNA
   <213> Canis lupus
<400> 95
   taaaggtttg gtcctagcct tcctattagt ttttagtaga cttacacatg caagcctcca      60
   cgccccagtg agaatgccct taaaatcacc agtgatctaa aggagcaggt atcaagcaca     120
   ctcttaagta gctcataaca ccttgctaag ccacaccccc acgggataca gcagtgataa     180
   aaattaagcc ataaacgaaa gtttgactaa gccatactaa atagggttgg taaatttcgt     240
   gccagccacc gcggtcatac gattaaccca aactaatagg cctacggcgt aaagcgtgtt     300
   caagatactt taacactaaa gttaaaactt aactaagccg taaaaagcta cagttatcat       360
   aaaataaacc acgaaagtga ctttataata atctgactac acgatagcta agacccaaac       420
   tgggattaga taccccacta tgcttagccc taaacataga taattttaca acaaaataat       480
   tcgccagagg actactagca atagcttaaa actcaaagga cttggcggtg ctttatatcc       540
   ctctagagga gcctgttcta taatcgataa accccgataa acctcaccac ctttcgctaa       600
   ttcagtctat ataccgccat cttcagcaaa ccctcaaaag gtagaacagt aagcacaatc       660
   attttacata aaaaagttag gtcaaggtgt aacttatgag gtgggaagaa atgggctaca       720
   ttttctaccc aagaacattt cacgaatgtt tttatgaaat taaaaactga aggaggattt       780
   agtagtaaat taagaataga gagcttaatt gaatagggcc atgaagcacg cacacaccgc       840
   ccgtcaccct cctcaagtaa taagacacaa ccataaccat attaacttaa ctaaaacaca       900
   agaggagaca agtcgtaaca aggtaagcat accggaaggt gtgcttggat taat             954
<210> 96
   <211> 980
   <212> DNA
   <213> Sus scrofa
<400> 96
   ttgcaaacac acagtaacag ctgcctgttc gtttagtctt aataaaatta cacatgcaag 60
   tatccgcgcc ccggtgagaa tgccctccag atcttaaaga tcaaaggagg cagttatcat 120
   tcacacttgc ttcgggagcg catcgccttg ctcaaccacc cccccacggg aaacagcagt 180
   gataaaaatt aagccatgaa cgaaagtttg actaagttat attaattaga gttggtaaat 240
   ctcgtgccag ccaccgcggt catacgatta acccaaattt atagatccac ggcgtaaaga 300
   gtgtttaaga aaaaaaaatc acaatagagt taaattataa ctaagctgta aaaagcccta 360
   gttaaaataa aataacccac gaaagtgact ctaataatcc tgacacacga tagctaggac 420
   ccaaactggg attagatacc ccactatgcc tagccctaaa cccaaatagt tacataacaa 480
   aactattcgc cagagtacta ctcgcaactg cctaaaactc aaaggacttg gcggtgcttc 540
   acatccacct agaggagcct gttctataat cgataaaccc cgatagacct taccaaccct 600
   tgccaattca gcctatatac cgccatcttc agcaaaccct aaaaaggaac aatagtaagc 660
   acaatcatag cacataaaaa cgttaggcaa ggtgtagctt atgggttgga aagaaatggg 720
   ctacattttc tacataagaa tacccaccat acgaaagttt ttatgaaact aaaaaccaaa 780
   ggaggattta gcagtaaatc aagaatagag tgcttgattg aataaggcca tgaagcacgc 840
   acacaccgcc cgtcaccctc ctcaagcatg tagtaataaa aataacctat attcaattac 900
   acaaccatgc aagaagagac aagtcgtaac aaggtaagca tactggaaag tgtgcttgga 960
   ttaccaaagc atagcataaa 980
<210> 97
   <211> 892
   <212> DNA
   <213> Gallus gallus
<400> 97
   aatgccccca aacctttctt cccaagcaaa aggagcaggt atcaggcaca ctcagcagta      60
   gcccaagacg ccttgcttaa gccacacccc cacgggtact cagcagtaat taaccttaag     120
   caataagtgt aaacttgact tagccatagc aacccagggt tggtaaatct tgtgccagcc     180
   accgcggtca tacaagaaac ccaaatcaat agctacccgg cgtaaagagt ggccacatgt     240
   tatctgcacc agctaagatt aaaatgcaac caagctgtca taagcctaag atccacctaa     300
   acccaaccca aatccatctt agcctcaacg attaatttta acccacgaaa gctaggaccc     360
   aaactgggat tagatacccc actatgccta gccctaaatc tagatacctc ccatcacaca     420
   tgtatccgcc tgagaactac gagcacaaac gcttaaaact ctaaggactt ggcggtgccc     480
   caaacccacc tagaggagcc tgttctataa tcgataatcc acgattcacc caaccacccc     540
   ttgccagcac agcctacata ccgccgtcgc cagcccacct ctaatgaaag aacaacagtg     600
   agctcaatag cccctcgcta ataagacagg tcaaggtata gcctatgggg tgggagaaat     660
   gggctacatt ttctaacata gaacaaacga aaaaggacgt gaaacccgcc cttagaagga     720
   ggatttagca gtaaagtgag atcatacccc ctaagctcac tttaagacgg ctctgaggca     780
   cgtacatacc gcccgtcacc ctcttcacaa gccatcaaca tcaataaata tatacttccc     840
   ctcccggcta aagacgaggc aagtcgtaac aaggtaagtg taccggaagg tg             892
<210> 98
   <211> 971
   <212> DNA
   <213> Meleagris gallopavo
<400> 98
   aaagacttag tcctaacctt actattgatt tttgctaaac atatacatgc aagtatccgc      60
   atgccagtga aaatgcccta accccttaag aaaagaataa aggagcaggt atcaggcaca     120
   ctctaatgta gcccaagacg ccttgcttga gccacacccc cacgggtatt cagcagtaat     180
   taaccttaag caataagtgt aaacttgact tagccatagc aactttaggg ttggtaaatc     240
   ttgtgccagc caccgcggtc atacaagaaa cccaaatcaa tagccatccg gcgtaaagag     300
   tggtcacatg ctatctatac caattaagat caaagtgtaa ctaagctgtc ataagcccaa     360
   gattcaccta agcccagcct aaaaaatgat cttaacttaa cgatcaattt aaagccacga     420
   aagccagggc acaaactggg attagatacc ccactatgcc tggccctaaa tcttgatact     480
   aatatactca cgtatccgcc tgagaactac gagcacaaac gcttaaaact ctaaggactt     540
   ggcggtgccc taaacccacc tagaggagcc tgttctgtaa tcgataatcc acgatccacc     600
   caaccacctc ttgccaacac agcctacata ccgccgtcgc cagcccacct aaaatgaaag 660
   atcaatagtg agctcaatag tcccactaac aagacaggtc aaggtatagc ccatgaggtg 720
   gaagaaatgg gctacatttt ctaacataga acagacgaaa aagggcgtga aactcgccct 780
   tggaaggagg atttagcagt aaagtaagac catacttctc ttaagcctac ttaaagacgg 840
   ccctggggca cgtacatacc gcccgtcacc ctcctcacaa gctatcaatt tcaataaata 900
   atacccaacc ctagctaaag atgaggtaag tcgtaacaag gtaagcgtac cggaaggtgc 960
   gcttagacta c 971
<210> 99
   <211> 989
   <212> DNA
   <213> Anser anser
<400> 99
   aaagacttag tcctaacctt acggttggtt tttgctaaat atatacatgc aagtatccgc 60
   gccccagtgt aaacgccctc gaccacctac ccccatacag gccttgagga gcgggtatca 120
   ggcacaccca agtagtagcc caagacgcct cgctaagcca cgcccccacg ggtattcagc 180
   agtaattaac attaagcaat gagtgcaaac ttgacttagt tatagcaaca gcctaacttc 240
   aagggttggt aaatcttgtg ccagccaccg cggtcataca agaaacccaa atcaaccgtc 300
   ctattgacac ggcgtaaaga gtggtaaaat gcctatccta gctaactaag atcaaaatgc 360
   aactgagctg tcataagccc aagatgcacc taaacacacc attaagatga tcttaggaac 420
   taacgactga tttaaaccca cgaaagccag ggcccaaact gggattagat accccactat 480
   gcctggccct aaatcttgat acttacttta ccgaagtatc cgccagagaa ctacgagcac 540
   aaacgcttaa aactctaagg acttggcggt gccccaaacc cacctagagg agcctgttct 600
   acaatcgata atccccgatt aacccaacca ccccttgcca acacagccta cataccgccg 660
   tcgccagccc acctcgaatg agagcacaac agtggacaca atagcacccc gctaataaga 720
   caggtcaagg tatagcctat ggagtggaag aaatgggcta cattccctat tcatagggca 780
   cacggaaaga agcgtgaaac cacttctgga aggcggattt agcagtaaag tgggacaata 840
   gagcctactt taagccggcc ctggggcacg tacataccgc ccgtcaccct cctcaaaagc 900
   cacatcccac ataactaata ccataaatac gctgaagatg aggtaagtcg taacaaggta 960
   agtgtaccgg aaggtgtact tagaatatc 989
<210> 100
   <211> 981
   <212> DNA
   <213> Anas platyrhynchos
<220>
   <221> misc-feature
   <222> (780)..(780)
   <223> n is a, c, g, or t
<400> 100
   aaagacttag tcctaacctt acagttggtt tttgctagac atatacatgc agtatccgcg      60
   ccccagtgat aaatgccctc aatagccttc accccaggcc ttaaggagcg ggtatcaggc     120
   acacccaagc agtagcccaa gacgccttgc taagccacgc ccccacgggt attcagcagt     180
   agttaacatt aagcaatgag tgcaaactcg acttagtcat agcaagcctc cacccaaggg     240
   tcggtaaatc ttgtgccagc caccgcggtc atacaagaga cccaaatcaa ctgtcctaca     300
   agcggcgtaa agagtggtaa gatgcctatc ctacctaact aagatcaaaa tgcaactaag     360
   ctgtcgcaag cacaagatgc acctaaacac accatcaaga tgatcttaga aactagcgat     420
   taatttgaac ccacgaaagc cagggcccaa actgggatta gataccccac tatgcctggc     480
   cctaaatctt gaacttaccc taccgaagta tccgccatat aactacgagc acaaacgctt     540
   aaaactctaa ggacttggcg gtgcctaaac ccacctagag gagcctgttc tgtaatcgat     600
   gatccacgat caacccaacc gccccttgcc gaacacagcc tacataccgc cgtcgccagc     660
   ccacctcgaa tgagagcgca acagtgggcg caacagcacc ccgctaataa gacaggtcaa     720
   ggtatagcct atgggacgga agaaatgggc tacattccct atgcataggg cagcacggan     780
   agaagtatga aactgcttct agaaggagga tttagcagta aagcgggaca ataaagctcg     840
   ctttaagccg gccctagggc acgtacatac cgcccgtcac cctcctcata agccacaccc     900
   ccacataact aataccacgt aaatgccaaa gatgaggtaa gtgtaacaag gtaagtgtac     960
   cggaaggtgt acttagaata c                                               981
<210> 101
   <211> 450
   <212> DNA
   <213> Equus caballus
<400> 101
   aaactgggat tagatacccc actatgctta gccctaaact aaaatagctt accacaacaa 60
   agctattcgc cagagtacta ctagcaacag cctaaaactc aaaggacttg gcggtgcttt 120
   acatccctct agaggagcct gttccataat cgataaaccc cgataaaccc caccatccct 180
   tgctaattca gcctatatac cgccatcttc agcaaaccct aaacaaggta ccgaagtaag 240
   cacaaatatc caacataaaa acgttaggtc aaggtgtagc ccatgggatg gagagaaatg 300
   ggctacattt tctaccctaa gaacaagaac tttaacccgg acgaaagtct ccatgaaact 360
   ggagactaaa ggaggattta gcagtaaatt aagaatagag agcttaattg aatcaggcca 420
   tgaagcgcgc acacaccgcc cgtcaccctc 450
<210> 102
   <211> 373
   <212> DNA
   <213> Felis catus
<400> 102
   gcttagccct aaacttagat agttacccta aacaaaacta tccgccagag aactactagc 60
   aatagcttaa aactcaaagg acttggcggt gctttacatc cctctagagg agcctgttct 120
   ataatcgata aaccccgata tacctcacca tctcttgcta attcagccta tataccgcca 180
   tcttcagcaa accctaaaaa ggaagaaaag taagcacaag tatcttaaca taaaaaagtt 240
   aggtcaaggt gtagctcatg agatgggaag caatgggcta cattttctaa aattagaaca 300
   cccacgaaga tccttacgaa actaagtatt aaaggaggat ttagtagtaa atttgagaat 360
   agagagctca att 373
<210> 103
   <211> 615
   <212> DNA
   <213> Bos taurus
<400> 103
   cagccttcct gttaactctt aataaactta cacatgcaag catctacacc ccagtgagaa        60
   tgccctctag gttattaaaa ctaagaggag ctggcatcaa gcacacaccc tgtagctcac       120
   gacgccttgc ttaaccacac ccccacggga aacagcagtg acaaaaatta agccataaac       180
   gaaagtttga ctaagttata ttaattaggg tttggtaaat ctcgtgccag ccaccgcggt       240
   catacgatta acccaagcta acaggagtac ggcgtaaaac gtgttaaagc accataccaa       300
   atagggttaa attctaacta agctgtaaaa agccatgatt aaaataaaaa taaatgacga       360
   aagtgaccct acaatagccg acgcactata gctaagaccc aaactgggat tagatacccc       420
   actatgctta gccctaaaca cagatattac ataaacaaaa ttattcgcca gagtactact       480
   agcaacagct taaactcaaa ggacttggcg gtgctttata tccttctaga ggagcctgtt       540
   ctataatcga taaaccccga taaacctcac caattcttgc taatacagtc tatataccgg       600
   catcttcaga aacct                                                        615

## Description

The invention as defined in the claims relates to the field of characterization of the meat content of any food sample based on any high throughput sequencing technology used to sequence any identical gene fragment (genomic or mitochondrial) amplified from universal animal primers and identified based on the high information polymorphic content between those common primers.

The invention as defined in the claims relates to the field of analytical methods that have been developed to identify and quantify the more frequent animal species present in ground meat, meat preparations and meat products and to quantify the proportion of them.
Species identification is a major concern due to the increased awareness of consumers regarding the composition of foods, and the need to verify labelling statements. Ground meat, meat preparations and processed meat products are susceptible targets for fraudulent labelling due to the economic profit that results from selling cheaper meats as partial or total replacement for high- valued meats. Several analytical methods are used for authentication of meat and meat products. The analytical methods include polymerase chain reaction, chromatography, mass spectrometry, microscopy, spectroscopy, electronic spin resonance, and enzymatic assays. In species determination, analysis of DNA and protein are common practices.
Among DNA-based methods, Polymerase Chain Reaction (PCR) is an effective technique that is highly accurate and relatively fast. Conventional qualitative PCR methods have a satisfactory performance in the qualitative detection of meat species. However, the need for methods that give quantitative results has arisen following the introduction of labelling obligations. Moreover, quantification is also necessary for the detection of intentional adulteration of meat products or accidental contamination in the production line. There is therefore a need for improved methods for the quantitative determination of the species origin(s) of meat and meat products.

The invention provides a method defined in the appended claims for determining the species origin(s) of DNA in a sample comprising conducting metagenomic sequence analysis of the sample to identify DNA from the present species and the relative proportions of them. This may be followed by species specific quantitative polymerase chain reaction (qPCR) to amplify and quantify a species specific target from the identified species DNA. In particular, the invention provides a method defined in the appended claims for determining the species origin(s) of DNA in a food sample comprising conducting metagenomic sequence analysis of the food sample by high throughput sequencing to identify DNA from at least one species, wherein the metagenomic analysis comprises amplification and sequencing of a metagenomic target DNA sequence which is part of the myostatin (MSTN) gene, said metagenomic target DNA sequence being selected to allow for: i) co-amplification of the metagenomic target DNA sequence from a plurality of species using a set of conserved, common primers comprising primers comprising or consisting of the sequence of SEQ ID NO:1, SEQ ID NO:2, and SEQ ID NO:3; and ii) the identification of species origin of any amplified metagenomic target DNA on the basis of its sequence; wherein the method comprises the step of amplifying target DNA using a set of primers comprising or consisting of the sequence of SEQ ID NO:1, SEQ ID NO:2, and SEQ ID NO:3.

Among new generation molecular technologies the metagenomic analysis has emerged as a powerful tool. Metagenomics applies a suite of genomic technologies and bioinformatics tools to directly access the genetic content of entire communities of organisms. This technology is commonly used to describe microbial community profiles of various ecosystems like seas, soils, rumen, faeces, but also to study complex microbiota of foodstuff. The invention applies the technique of metagenomics in the first stage of a sequential identification, and proportion estimation of animal species in a sample or quantitation of the origin of DNA in a sample.
- Preferably the metagenomic target DNA sequence is sequenced by a process for massively parallel sequencing. In some embodiments, the process for massively parallel sequencing is selected from emulsion sequencing, and solid phase sequencing. The metagenomic DNA target sequence may be a chromosomal or mitochondrial gene conserved among all the animal species. The targeted gene has to be characterized both by conserved sequences usable for universal primers design and by a within primer sequence of lower conservation riche in polymorphisms within the various homologues of the species of interest, such that the specific sequence of the amplified target, between the primer sequences, may be used to identify the species of origin. As the mammalian mitochondria exists in multiple copies per cell, any mitochondrial DNA region (mtDNA) could be used for detection and identification of DNA traces (ex: absence of pork meat in Halal market). The chromosomal gene present in one copy per cell is suitable for the identification and estimation of relative proportion of species in meat products.

In the optional second stage of the method defined in the appended claims of the invention, real-time, or quantitative PCR (qPCR) is used to quantitate the amount of DNA of the species identified in the first stage as being present in the sample. Real-time PCR has demonstrated the highest improvement among PCR-based quantitative methods in recent years. In real-time PCR, the exponential amplification of target- specific DNA is monitored by an increased fluorescence signal. If the species under study is present in the matrix, a specific signal can be observed. As well as meeting the need for quantitative determination in meat species, this technique also has other advantages like higher sensitivity and specificity.
Quantitative PCR may be based on the use of intercalating dyes, or the use of a labelled reporter primer and/or probe. In some embodiments of the invention, therefore, the qPCR step is selected from qPCR using an intercalating dye and qPCR using a labelled reporter primer or probe. In embodiments based on intercalating fluorescent dyes, the dye may be SYBRGreen or EvaGreen. Such methods require the greatest sequence specificity due to the fact that these types of dyes bind to all double-stranded DNA present, including any nonspecific PCR products and the primer-dimer complex.

In other, preferable embodiments, the real-time quantitative PCR procedure for species identification is generally based on the use of a labelled reporter primer or probe. Preferably, the labelled reporter primer or probe is part of a fluorescence/quencher reporter system or other fluorescence reporter system in which amplification of the species specific DNA target results in a reduction or elimination of fluorescence quenching, or other increase in fluorescence, to result in a detectable fluorescent signal. For example a TaqMan fluorogenic probe (also referred to herein as a hydrolysis probe (the 5' to 3' exonuclease activity of the polymerase degrades the probe that has annealed to the template. Degradation of the probe releases the fluorophore from it and breaks the close proximity to the quencher, thus relieving the quenching effect and allowing fluorescence of the fluorophore)) may be used as the reporter system. This method uses an additional oligonucleotide, which is also bound specifically to the target DNA sequence during the annealing step. This oligonucleotide has both a reporter fluorescent dye and a quencher dye attached. The accumulation of specific PCR products is detected by hybridization and cleavage of a double- labelled fluorogenic probe during the amplification reaction.

Other alternative reporter systems may be employed. For example, the reporter system may comprise a molecular beacon probe, a pair of dual hybridization probes, an amplifluor primer system, a scorpion primer system, a light-up on- extension system or a QZyme primer system. Other suitable systems will be know to the skilled person and may be used to provide quantitative realtime PCR reporting.

Any suitable target sequences may be used for either or both stages of the process. To be suitable for stage 1 of the process (metagenomic analysis) the target must allow for co- amplification from a plurality of species using a set of conserved, common primers. Preferably a single pair of primers (one forward primer and one reverse primer) is used to co-amplify the metagenomic target DNA sequence from substantially all species of interest. However, the process is not limited to the use of just a single pair of primers. Metagenomic analysis may be improved, and expanded in scope by the addition of multiple forward and/or reverse primers which can allow for a broader range of species specific sequences that can be amplified. Multiple primers can also be used to ensure that amplification efficiency is broadly similar for all species of interest in order to eliminate or reduce over-representation of one species, possibly at the expense of another, in the amplification process.
The metagenomic target sequence is preferably a sequence which has the same number of copies per genome in each of the species suspected as being present in the food sample, so as to allow for a determination of the relative proportions of the different identified DNAs by the metagenomic analysis.
The skilled person is able to determine appropriate primer pairs for the metagenomic analysis, based upon an examination of a line-up of sequences from a homologous gene shared by the species of interest. Highly conserved regions of the gene of interest can be examined for the possibility of providing a relatively small number of primers capable of amplifying target from a large number of species. Preferably a small number of primers, for example 2 or 3 primers may be adequate to amplify a plurality of target sequences from e.g. at least 12 or 13 or more species. Primers may include one or more degenerate nucleotide bases in order to increase the range of species whose target DNA may be amplified in the metagenomic analysis. Primers may also be modified by the addition of adaptors or tags, or universal sequences for use in manipulation of amplicon products, including sequencing of the products.

In the metagenomic analysis, following amplification of target sequences, the amplicons so produced are sequenced to determine species origin. A further requirement, therefore, of the metagenomic target DNA sequence is that it allows for the identification of species origin of any amplified metagenomic target DNA on the basis of its sequence. This may be achieved by providing PCR primers from highly conserved regions (as discussed above), which flank regions of lower conservation amongst the various homologues of the species of interest, such that the specific sequence of the amplified target, between the primer sequences, may be used to identify the species of origin.

### Identification of animal species by NGS technology using chromosomal gene:

The inventors have found that the myostatin (MSTN) gene is a suitable gene for use in the process of the invention, particularly in the identification and quantitation of species in meat products. The MSTN gene encodes the myostatin protein (also known as growth differentiation factor 8 or GDF- 8). Myostatin is a secreted growth differentiation factor that is a member of the TGF beta protein family that inhibits muscle differentiation and growth in the process known as myogenesis. The gene is classified as a housekeeping gene, that is a group of genes coding mainly for essential proteins in the cellular basic function. These genes include conserved area useful for conserved primers design and variable area required for species identification and present in single copy in the genome, an important feature for the quantification method.

In all embodiments the myostatin gene is used in the invention defined in the appended claims to identify and quantitate DNA from at least one species from a genus selected from *Sus, Gallus, Bos, Ovis, Equus, Meleagris, Felis, Canis canis, capreolus capreolus, O.cuniculus, roe dear, Rabbit, Kangaro, Rattus rattus, mus musculus, Capra, Antilocapra, Cervus, Anas, Anser and Coturnix, for example, Sus scrofa, Gallus gallus, Bos Taurus, Ovis aries, Equus caballus, Meleagris gallopavo, Felis catus, Capra hircus, Antilocapra Americana, Cervus elaphus, Anas platyrhyncho, Anser anser and Coturnix chinensi.* In some embodiments, the metagenomic target DNA sequence is therefore from the MSTN gene. Preferably, the metagenomic target DNA sequence is amplified by a set of conserved primers comprising at least a primer comprising the sequence of SEQ ID NO. 1,SEQ ID NO. 2, and a primer comprising the sequence of SEQ ID NO. 3. In some embodiments the metagenomic target DNA sequence is amplified by a set of conserved primers comprising a primer comprising the sequence of SEQ ID NO. 1 and a primer comprising the sequence of SEQ ID NO. 2 and a primer comprising the sequence of SEQ ID NO. 3. In some embodiments, the primers consist of the sequences of SEQ ID NO. 1, SEQ ID NO. 2 and SEQ ID NO. 3. In some embodiments the primers having or comprising the sequences of SEQ ID No's 1 , 2 or 3 are further modified by the addition of adaptors, tags or universal sequences depending of the NGS technology used.

In some embodiments the set of primers for amplification of the metagenomic target DNA comprises a set of forward primers having the sequences of SEQ ID NO.s 4 and 5, and a set of reverse primers having the sequences of SEQ ID NO.s 6 to 13.

The MSTN gene is also suitable for use in the optional second stage of the process of the invention, that is the qPCR stage. Accordingly, in some embodiments, the species specific target DNA sequence is amplified by a set of primers and the resulting amplicon is detected by a labelled hybridisation probe, the primers and probes being selected from the following:
a) primers comprising or consisting of the sequences of SEQ ID NOS. 17 and 18 and a probe comprising or consisting of the sequence of SEQ ID NO. 19 for the quantitation of DNA sequences from pork (Sus scrofa); or
b) primers comprising or consisting of the sequences of SEQ ID NOS. 20 and 16 and a probe comprising or consisting of the sequence of SEQ ID NO. 21 for the quantitation of DNA sequences from chicken (Gallus gallus); or
c) primers comprising or consisting of the sequences of SEQ ID NOS. 22 and 16 and a probe comprising or consisting of the sequence of SEQ ID NO. 23 for the quantitation of DNA sequences from beef (Bos taurus); or
d) primers comprising or consisting of the sequences of SEQ ID NOS. 24 and 16 and a probe comprising or consisting of the sequence of SEQ ID NO. 25 for the quantitation of DNA sequences from sheep (Ovis aries); or
e) primers comprising or consisting of the sequences of SEQ ID NOS. 26 and 27 and a probe comprising or consisting of the sequence of SEQ ID NO. 28 for the quantitation of DNA sequences from horse (Equus caballus); or
f) primers comprising or consisting of the sequences of SEQ ID NOS. 29 and 16 and a probe comprising or consisting of the sequence of SEQ ID NO. 30 for the quantitation of DNA sequences from turkey (Meleagris gallopavo); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample.

Preferably, the primers and probes consist of the sequences of SEQ ID NOs. 14 to 32 respectively.

In some embodiments, the forward primers selected for the various species may include primers with some degeneracy and/or may include a mixture of primers. Conveniently, for the quantitation of DNA sequences from chicken, beef, sheep or turkey, the forward primer noted above may be replaced by a mixture of primers having the sequences of SEQ ID NO.s 14 and 15.

In preferred embodiments, the quantitation of species specific target DNA sequences is compared to a control or reference target sequence. Preferably the control or reference target sequence is part of the same gene as the species specific target sequence. In some embodiments, therefore, a control or reference target sequence is part of the MSTN gene sequence. In some embodiments the control or reference target sequence is co-amplified with the species specific target sequence, and is detected in real time (qPCR). In some embodiments a control or reference target sequence is amplified using a set of primers comprising or consisting of the sequences of SEQ ID NOs 14, 15 and 16, and detected using a probe comprising or consisting of the sequence of SEQ ID NO. 31 or SEQ ID NO. 32.

In another aspect, the invention provides the use defined in the appended claims of the primers as discussed above (comprising or consisting of the sequence of SEQ ID NOs 1, 2 and 3) with the primers and probes also discussed herein (comprising or consisting of the sequences of SEQ ID NOs 14 to 32) for the sequential metagenomic identification and relative proportion determination and species specific quantitation of DNA in a food sample, the selection of species specific primers and probes employed for quantitation being chosen on the basis of the metagenomic identification of species DNA present in the food sample. Preferably the sample is a meat sample.

In a further aspect, the invention provides a kit defined in the appended claims for metagenomic identification of DNA present in a food sample, comprising a set of primers for the metagenomic amplification of a metagenomic target sequence in the myostatin gene, said metagenomic target sequence being selected to allow for: i) co-amplification of the metagenomic target DNA sequence from a plurality of species using a set of conserved, common primers comprising primers comprising or consisting of the sequence of SEQ ID NO: 1, SEQ ID NO:2 and SEQ ID NO: 3; ii) the identification of species origin of any amplified metagenomic target DNA on the basis of its sequence; and optionally iii) the relative proportions between the different identified DNA. In some embodiments the metagenomic target sequence is selected to allow for amplification of DNA from at least 12 or 13 different species using a set of conserved, common primers. In some embodiments the set of conserved, common primers includes no more than three primers, for example two or three primers.

The set of primers of the kit comprises primers comprising the sequence of SEQ ID NO. 1 SEQ ID NO. 2 and a primer comprising the sequence of SEQ ID NO. 3. In some embodiments, the set of primers consists of three primers comprising the sequences of SEQ ID NO. 1, SEQ ID NO. 2 and SEQ ID NO. 3 respectively. Preferably, the primers consist of the sequences of SEQ ID NO. 1, SEQ ID NO. 2 and SEQ ID NO. 3. Any one or more of the primers may be adapted by the addition of an adaptor, tag or universal sequence. The invention further provides a kit defiened in the appended claims for quantitative PCR analysis of a species specific target sequence in a food sample, said kit comprising a set of primers and probes for the amplification and realtime detection of a sequence from the myostatin gene, said sequence being selected to allow for species specific amplification and/or detection. In some embodiments, the species specific amplification and/or detection can distinguish between species from a genus selected from *Sus, Gallus, Bos, Ovis, Equus, Meleagris, Felis, Capra, Antilocapra, Cervus, Anas, Anser and Coturnix, for example, Sus scrofa, Gallus gallus, Bos Taurus, Ovis aries, Equus caballus, Meleagris gallopavo, Felis catus, Capra hircus, Antilocapra Americana, Cervus elaphus, Anas platyrhyncho, Anser anser and Coturnix chinensis.*

In preferred embodiments, the set of primers and probes is selected from at least one of:
a) primers comprising or consisting of the sequences of SEQ ID NOS. 17 and 18 and a probe comprising or consisting of the sequence of SEQ ID NO. 19 for the quantitation of DNA sequences from pork (Sus scrofa); or
b) primers comprising or consisting of the sequences of SEQ ID NOS. 20 and 16 and a probe comprising or consisting of the sequence of SEQ ID NO. 21 for the quantitation of DNA sequences from chicken (Gallus gallus); or
c) primers comprising or consisting of the sequences of SEQ ID NOS. 22 and 16 and a probe comprising or consisting of the sequence of SEQ ID NO. 23 for the quantitation of DNA sequences from beef (Bos taurus); or
d) primers comprising or consisting of the sequences of SEQ ID NOS. 24 and 16 and a probe comprising or consisting of the sequence of SEQ ID NO. 25 for the quantitation of DNA sequences from sheep (Ovis aries); or
e) primers comprising or consisting of the sequences of SEQ ID NOS. 26 and 27 and a probe comprising or consisting of the sequence of SEQ ID NO. 28 for the quantitation of DNA sequences from horse (Equus caballus); or
f) primers comprising or consisting of the sequences of SEQ ID NOS. 29 and 16 and a probe comprising or consisting of the sequence of SEQ ID NO. 30 for the quantitation of DNA sequences from turkey (Meleagris gallopavo); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample.

In some embodiments of the kit primers having the sequences of SEQ ID NOs 20, 22, 24 and/or 29 may be replaced by primers comprising or consisting of the sequences of SEQ ID NOs 14 and 15.

In some embodiments the kits further contain a probe having a sequence of SEQ ID NO. 31 or SEQ ID NO. 32, which acts as a probe for a control or reference sequence in the MSTN gene.

The invention further provides a kit defined in the claims having some or all of the features recited above for metagenomic analysis and further having some or all of the features recited above for qPCR analysis. Optionally the kit may further comprise any reagent suitable for use in the metagenomic or qPCR steps of the process of the invention.

The invention will now be described in further detail with reference to the following examples and figures, in which:
Figure 1 shows an alignment of myostatin Exon1 from 12 mammalian and bird species (frequently found in meat and meat products).
Figure 2 shows the binding site of primers and probes designed on myostatin exon 1 for pork, beef, horse, sheep, chicken and turkey. (1) represents Forward primer; (3) : reverse primer; (2):Taqman probe
Figure 3 shows an amplification curve (A) and linear regression (B) of real-time PCR detection for beef.
Figure 4 shows metagenomic analysis of meat samples
Figure 5 shows relative proportion of mammals and poultry composition in 20 DNA mixes using myostatin as target gene. The table below include all the sequences analysed and identified as belonging to one of the 8 animal species (all the numbers <100 are not representative). Those results are not corrected for the genome size explaining the difference to the expected 50-50 % or 25-25-25-25 % of different species.
Figure 6 shows relative proportion of 14 mammals and poultry identified by Next generation sequencing.

Identification of animal species by NGS technologie using mitochondrial gene.

The mitochondrial gene coding for the ribosomal 12S ribosomal RNA is known for his impact in the higher eukaryotes taxonomy. These genes include conserved area useful for conserved primers design and variable area required for species identification. Inside the higher eukaryotic cell the mitochondria number vary between 10 and 1000 units. This target gene could be chosen to detect and identify tiny DNA traces of animal species.

### Material and methods

### DNA isolation

Twenty-five gram sample of meat or meat product was homogenized for 1 minute in 225 ml in a tempo bag (bioMerieux Basingstoke, England, ref 80015) with sterile physiologic water using a stomacher apparatus. 1.5 ml of the suspension was then used for total genomic DNA extraction using DNeasy Blood & Tissue Kit (QIAGEN, Crawley, UK). The DNA concentrations were evaluated by fluorometric ds DNA quantification using PicoGreen.

### Metagenomic analysis using MSTN gene

### Conserved primers design for pyrosequencing

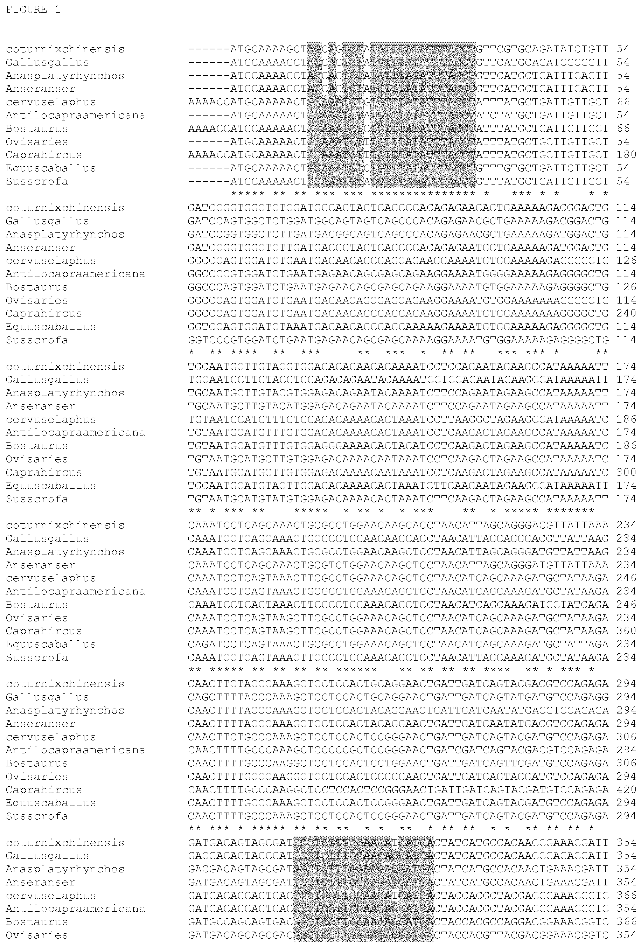

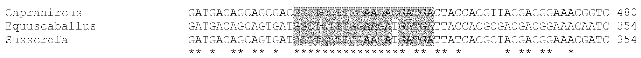

The myostatin exon 1 sequence from 12 frequent species present in meat products *(Sus scrofa, Bos taurus, Gallus gallus, Equus caballus, Ovis aries, Capra hircus, Anas platyrhynchos, Anser anser and Coturnix chinensis, Cervus elaphus, Antilocapra Americana),* were aligned to compare conserved area used for the conserved primers design (fig. 1). 3 primers are designed to amplified a PCR fragment of 313 bases (MSTN-E1-UP1 :AGTCTATGTTTATATTTACCT (SEQ ID NO. 1), MSTN-E1-UP2 :AATCTYTGTTTATATTTACCT (SEQ ID NO. 2), MSTN-E1- DN :TCATCRTCTTCCAARGAGCC (SEQ ID NO. 3)). The amplified fragment shows a sequence diversity sufficient to identify species by sequencing. The oligonucleotide design included 454 Life Sciences A or B sequencing titanium adapters (Roche Diagnostics, Vilvoorde, Belgium) and multiplex identifiers (MIDs) fused to the 5' end of reverse primer.

### ribosomal 12S RNA:

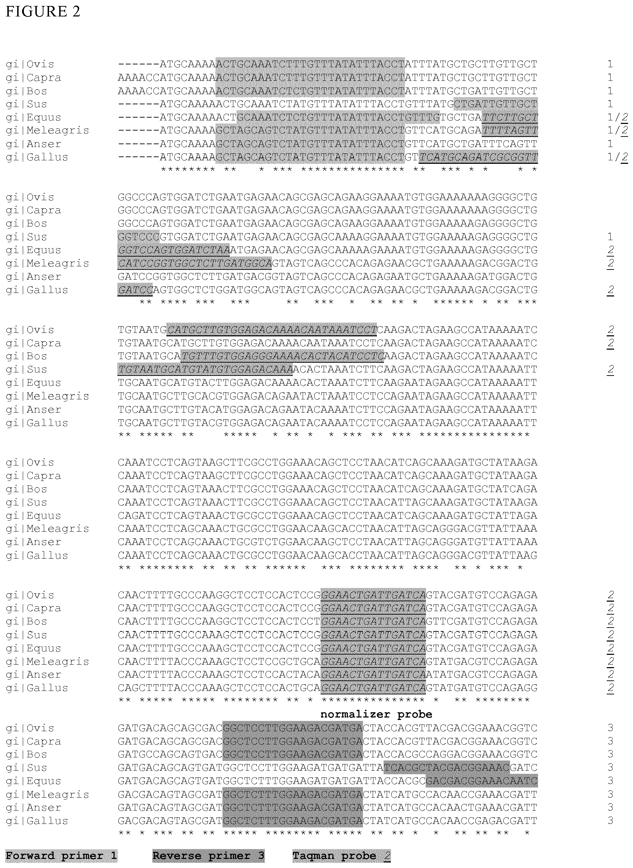

The ribosomal 12S RNA sequences from 13 frequent species present in meat products were aligned to design conserved primers (fig 2). 3 primers are designed to amplified a PCR fragment of 514 bases (NGS12S-FW1 : 5'-AYACCGCCCGTCACCCTC-3 '; NGS12S-REV15'-
TWGGCTTTTCACCTCTAC - 3'; NGS12S-REV2: 5'- TWGGCATGTCACCTCTAC - 3 ')

**FORWARD PRIMERS REVERSE PRIMERS**

| | | |
|---|---|---|
| EquusCaballus | ACACCGCCCGTCACCCTC | GTACCGTAAGGGAACGATGA |
| FelisCatus | ACACCGCCCGTCACCCTC | GTACCGTAAGGGAACGATGA |
| BT1 | ACACCGCCCGTCACCCTC | GTACCGCAAGGGAACGATGA |
| BT2 | ACACCGCCCGTCACCCTC | GTACCGCAAGGGAACGATGA |
| CapraHircus | ACACCGCCCGTCACCCTC | GTACCGTAAGGGAATGATGA |
| CanisLupusfamiliaris | ACACCGCCCGTCACCCTC | GTACCGTAAGGGAATGATGA |
| SS-Pietrain | ACACCGCCCGTCACCCTC | GTACCGTAAGGGAAAGATGA |
| SS-LW | ACACCGCCCGTCACCCTC | GTACCGTAAGGGAAAGATGA |
| GallusGallusl | ATACCGCCCGTCACCCTC | GTACCGCAAGGGAAAGATGA |
| GallusGallus2 | ATACCGCCCGTCACCCTC | GTACCGCAAGGGAAAGATGA |
| Meleagris Gallopavo | ATACCGCCCGTCACCCTC | GTACCGTAAGGGAAAGATGA |
| AnserAnser | ATACCGCCCGTCACCCTC | GTACCGTAAGGGAAAGATGA |
| AnasPlatyrhynchos | ATACCGCCCGTCACCCTC | GTACCGTAAGGGAAAGATGA |

Conserved sequences observed and used for common primers design after ribosomal 12S RNA sequences alignment of 13 frequent mammalian and bird species.

The 2 amplified fragments show a sequence diversity sufficient to identify species by sequencing. The oligonucleotide design included 454 Life Sciences A or B sequencing titanium adapters (Roche Diagnostics, Vilvoorde, Belgium) and multiplex identifiers (MIDs) fused to the 5' end of reverse primer. These amplicons may be used with or without NGS technology.

A full set of primers designed for 454 Life Sciences sequencing is as follows:
**Fusion FW (B adaptator)** - (MSTN-E1-UP)
   MSTNE1-UP1:5'-CCTATCCCCTGTGTGCCTTGGCAGTCTCAGAGTCTATGTTTATATTTACCT-3' (SEQ ID NO. 4)
   MSTNE1-UP2:5'-CCTATCCCCTGTGTGCCTTGGCAGTCTCAGAATCTYTGTTTATATTTACCT-3' (SEQ ID NO. 5)
**Fusion Rev : (A adaptator) -Key-MID-** (MSTN-E1-DN)
   MSTNE1-DN-MID130 :5'-CCATCTCATCCCTGCGTGTCTCCGACTCAGAGACTGCACTCATCRTCTTCCAARGAGCC-3 (SEQ ID NO. 6)
   MSTNE1-DN-MID101 :5'-CCATCTCATCCCTGCGTGTCTCCGACTCAGATCGCAGGCTCATCRTCTTCCAARGAGCC-3 (SEQ ID NO. 7)
   MSTNE1-DN-UM102 :5'-CCATCTCATCCCTGCGTGTCTCCGACTCAGACGCTCGACATCATCRTCTTCCAARGAGCC-3 (SEQ ID NO. 8)
   MSTNE1-DN-UM103 :5'-CCATCTCATCCCTGCGTGTCTCCGACTCAGAGACGCACTCTCATCRTCTTCCAARGAGCC-3 (SEQ ID NO. 9)
   MSTNE1-DN-UM104 :5'-CCATCTCATCCCTGCGTGTCTCCGACTCAGAGCACTGTAGTCATCRTCTTCCAARGAGCC-3 (SEQ ID NO. 10)
   MSTNE1-DN-UM105 :5'-CCATCTCATCCCTGCGTGTCTCCGACTCAGATCAGACACGTCATCRTCTTCCAARGAGCC-3 (SEQ ID NO. 11)
   MSTNE1-DN-UM106 :5'-CCATCTCATCCCTGCGTGTCTCCGACTCAGATATCGCGAGTCATCRTCTTCCAARGAGCC-3 (SEQ ID NO. 12)
   MSTNE1-DN-UM107 :5'-CCATCTCATCCCTGCGTGTCTCCCACTCAGCGTGTCTCTATCATCRTCTTCCAARGAGCC-3 (SEQ ID NO. 13)

These amplicons may be used with our without NGS technology.

### For ribosomal 12S RNA gene.

**Fusion FW : (B adaptator)-key-MID-(NGS12S-FW1)**
   NGS12S-FW MID 100: 5'-
   NGS12S-FW MID101: 5'-
   NGS12S-FW MID102:.5'-
**Fusion Rev: (A adaptator)-key-(NGS12S-REV1)**
   NGS12S-REV1.1 : 5'-CCTATCCCCTGTGTGCCTTGGCAGTCTCAGTWGGCTTTTCACCTCTAC-3'
   NGS12S-REV1.2 :_5'-CCTATCCCCTGTGTGCCTTGGCAGTCTCAGTWGGCATGTCACCTCTAC-3'
   NGS12S-REV1.3 :_5'-CCTATCCCCTGTGTGCCTTGGCAGTCTCAGTWGGCWTKTCACCTCTA-3'

These amplicons may be used with or without NGS technology.

### MSTN gene library construction and pyrosequencing

The amplification mix contained 5 U of FastStart high fidelity polymerase (Roche Diagnostics, Vilvoorde, Belgium), lx enzyme reaction buffer, 200 µM dNTPs (Eurogentec, Liege, Belgium), 0.2 µM of each primer and 100 ng of genomic DNA in a volume of 25 µl. Thermocycling conditions consisted of a denaturation at 94°C for 10 min followed by 25 cycles of 94°C for 30 s, 50°C for 30 s, 72°C for 1 min and a final elongation step of 7 min at 72°C. These amplifications were performed on an Ep Master system gradient apparatus (Eppendorf, Hamburg, Germany). The PCR products of approximately 313 bp were checked by gel electrophoresis and purified using the AMPure kit (Agencourt Bioscience Corporation, Beverly, USA) to remove amplicons shorter than 100 bp. Equal amounts of each of the PCR products were pooled and subsequently amplified by emulsion PCR before sequencing. Pyrosequencing was performed with the Roche 454 GS Junior Sequencer (Roche, Basel, Switzerland).

### Bioinformatic analysis

Image and data processing for amplicon sequencing was performed using the Genome Sequencer FLX System Software Package 2.3 (Roche, Basel, Switzerland). The Mothur program was used for the first part of the sequences analysis (SCHLOSS et al., 2009). The shorter sequences were discarded. The sequences with ambiguous bases or homopolymers longer than ten nucleotides and with an average quality score lower than 25 were removed along with tag and primer sequences. Sequences from multiplexed samples were assigned based on the presence of the unique barcodes assigned to each sample. A distance matrix was prepared, (distance= 0.01) and the sequences were clustered to operational taxonomic units (OTUs). The representative sequences of each OTU were compared to the NCBI database using the Basic Local Alignment Search Tool (BLASTN) a private database of in-house sequences.

### Genbank myostatin gene sequences were:

- 5178958 ref |NM_21445.2 Sus scrofa myostatin (MSTN), mRNA
- 241897522 gb GQ184147.1 Bos taurus myostatin mRNA, complete cds
- 4782570 ref |NM_001001461.1 Gallus gallus myostatin (MSTN), mRNA
- 12652492 ref |NM_001081817.1 Equus caballus myostatin (MSTN), mRNA
- 57164246 ref |NM_001009428.1 Ovis aries myostatin (MSTN), mRNA
- 289600017 gb GQ246166.1 Capra hircus myostatin precursor, mRNA
- 17064128 gb AF440861.1 Anas platyrhynchos myostatin (MSTN) mRNA,
- 1706410 gb AF440862.1 Anser anser myostatin (MSTN) mRNA
- 14984905 gb EF62955.1 Cervus elaphus xanthopygus myostatin mRNA
- 482746 gb AY62909.1 Antilocapra americana myostatin mRNA
- 1706414 gb AF440864.1 Coturnix chinensis myostatin (MSTN) mRNA
- 512867089 : 168-1181 Xenopus (Silurana) tropicalis myostatin (mstn), mRNA
- 410969071 ref XM_00990972.1 Felis catus myostatin (MSTN), mRNA

Sequences shown in the sequence listing numbered 80 to 93 inclusive are myostatin sequences from various animals from genbank.

Sequences shown in the sequence listing numbered 94 to 103 inclusive are mitochondrial 12S sequences from various animals from genbank.

### Quantification by qPCR using MSTN gene

### Oligonucleotides primers and probes

A TaqMan-based real-time Polymerase Chain Reaction (qPCR) assay was developed for quantify 6 frequent species present in meat products (Sus scrofa, Bos taurus, Gallus gallus, Equus caballus, Ovis aries and Meleagris gallopavo). By aligning the myostatin Exon 1 DNA sequence of these species according to the NCBI database forward and reverse primers and Taqman probes were designed for each meat-specific region (fig.2). The list of these primers and probes is shown in Table 1.

**Table 1: Sequences of primers and probes used in this study.**

| | | **sequence** |
|---|---|---|
| | forward primer | 5'-CTGATTGTTGCTGGTCCC-3' |
| **pork** | reverse primer | 5'-GTTTCCGTCGTAGCGTGA-3' |
| | Taqman probe | 5'-(FAM)-TGTAATGCATGTATGTGGAGACAAA-(BHQ)-3' |
| | forward primer | 5'-GCAGCAGTCTATGTTTATATTTACCT3' |
| **chicken** | reverse primer | 5'-TCATCRTCTTCCAARGAGCC-3' |
| | Taqman probe | 5'-(FAM)-TTCATGCAGATCGCRGTTGATCC-(BHQ)-3' |
| | forward primer | 5'-ACTGCAAATCTCTGTTTATATTTACCT-3' |
| **beef** | reverse primer | 5'-TCATCRTCTTCCAARGAGCC-3' |
| | Taqman probe | 5'-(FAM)-TGTTTGTGGAGGGAAAACACTACATCCTC-(BHQ)-3' |
| | forward primer | 5'-ACTGCAAATCTTTGTTTATATTTACCT-3' |
| **sheep** | reverse primer | 5'-TCATCRTCTTCCAARGAGCC-3' |
| | Taqman probe | 5'-(FAM)-CATGCTTGTGGAGACAAAACAATAAACCT-(BHQ)-3' |
| | forward primer | 5'-GCAAATCTCTGTTTATATTTACCTGTTTG-3' |
| **horse** | reverse primer | 5'-GGTAATGATTGTTTCCGTCGTC-3' |
| | Taqman probe | 5'-(FAM)-TTCTTGCTGGTCCAGTGGATCTAA-(BHQ)-3' |
| | forward primer | 5'-GCTAGCAGTCTATGTTTATATTTACCT-3' |
| **turckey** | reverse primer | 5'-TCATCRTCTTCCAARGAGCC-3' |
| | Taqman probe | 5'-(FAM)-TGCAGATTTTATGTTCATCCGGT-(BHQ)-3' |
| | forward primer | 5'-GCTAGCAGTCTATGTTTATATTTACCT-3' |
| **normalizer control** | reverse primer | 5'-TCATCRTCTTCCAARGAGCC-3' |
| | Taqman probe | 5'-(FAM)-GGAACTGATTGATCA-(BHQ)-LNA-3' |

The sequences in Table 1 have the following SEQ ID NOs:
Pork forward primer: SEQ ID NO. 17. Pork reverse primer: SEQ ID NO.18. Pork Taqman probe: SEQ ID NO. 19.
Chicken forward primer: SEQ ID NO. 20. Chicken reverse primer: SEQ ID NO.16. Chicken Taqman probe: SEQ ID NO. 21.
Beef forward primer: SEQ ID NO. 22. Beef reverse primer: SEQ ID NO.16. Beef Taqman probe: SEQ ID NO. 23.
Sheep forward primer: SEQ ID NO. 24. Sheep reverse primer: SEQ ID NO. 16. Sheep Taqman probe: SEQ ID NO. 25.
Horse forward primer: SEQ ID NO. 26. Horse reverse primer: SEQ ID NO.27. Horse Taqman probe: SEQ ID NO. 28.
Turkey forward primer: SEQ ID NO. 29. Turkey reverse primer: SEQ ID NO.30. Turkey Taqman probe: SEQ ID NO. 31.
Normalizer forward primer: SEQ ID NO. 14. Normalizer reverse primer: SEQ ID NO. 16.

Normalizer Taqman probe: SEQ ID NO. 31. A further normalizer Taman probe that may be use is:
FAM-AGTGGAGGAGCYTTGGGYAAAAG-BHQ (SEQ ID NO. 32)

The forward primers for chicken, beef, sheep and turkey noted above may be replaced by a mixture of the following two primers:
GCTAGCAGTCTATGTTTATATTTACCT (SEQ ID NO. 14)
ACTGCAAATCTYTGTTTATATTTACCT (SEQ ID NO. 15).

### Conventional PCR protocol

Real-time PCR was performed using the LightCycler 480 system (Roche, Basel, Switzerland). The PCR reaction mixtures were placed in a 12 µl final volume containing 6 µl of LC480 probe master mix (Roche, Basel, Switzerland), 2 µl of template DNA (at 5ng/µl), 0,25 µl of primer pair (10µM each), 0,125 µl of Taqman probe (10µM). The reaction conditions included the initiation step for 10 min at 95°C, followed by 40 cycles of 15s at 95°C and 1 min at 60°C. The real-time system is supplied with the Lightcycler 480 Software version 1.5 using unique Roche algorithms for highly accurate and robust automated data analysis

### Specificity and sensitivity test.

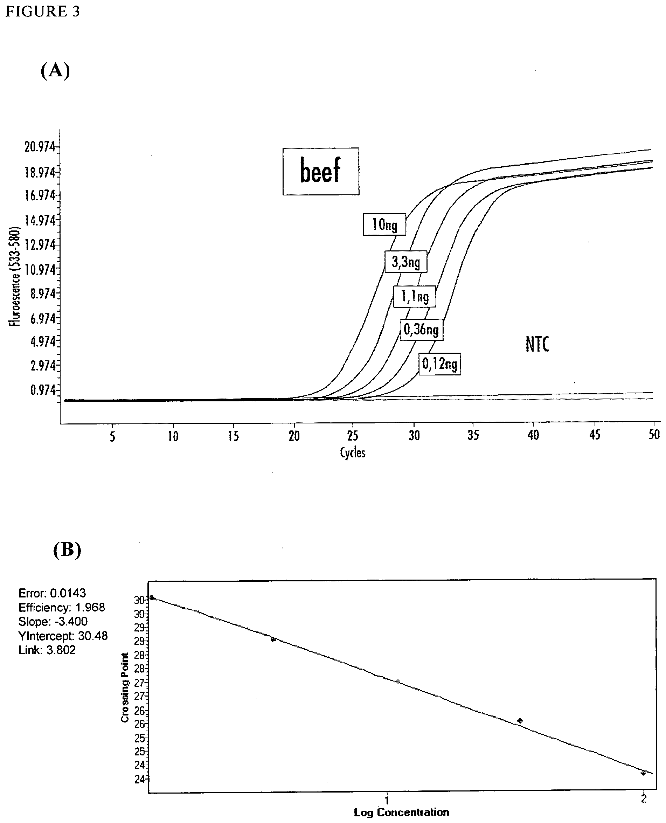

The specificity of each species test was confirmed by the amplification of 100ng of chicken, turkey, horse, donkey, pork, bovine and ovine genomic DNA and a negative control without DNA (NTC). Each assay was tested against DNA from all six species, against its target species and the 5 remaining species to confirm specificity. In sensitivity test of the specific primers and probes, PCR amplifications were examined between specific primers and 3-fold serial dilutions of DNAs isolated from the meat of each target species ranging from lOng to 0,4 ng. The standard curves for all the 6 detection systems were constructed by using the Cp value obtained from the corresponding DNA concentration (fig 3).

### Results

In this study, metagenomic and real-time PCR assays based on the amplification of same fragment of genomic myostatin gene were developed and evaluated for detection and quantification of 6 frequent species present in meat and meat products (pork, beef, horse, sheep, chicken and turkey).

### Metagenomic analysis using MSTN gene

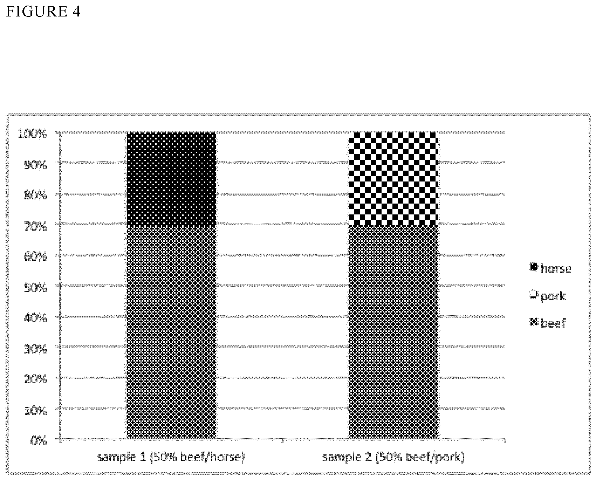

The metagenomic assay was applied to two commercial meat preparations, the first composed of 50% of beef and 50% of pork meat, the second composed of 50% of beef and 50% of horse meat. The treated sequences were compared to the NCBI database using the Basic Local Alignment Search Tool (fig. 4). The metagenomic analysis allowed identification of all the meat components but the proportions are not fully representative. The proportion of beef are more important in the two samples tested to the detriment of the 2 others species. To obtain more quantitative analysis its important to avoid competition between meat components thus to analyse in the early part of the exponential phase because of all reagents are still in excess and the species which is present in a low amount will not compete with the others.

### Quantification by qPCR using MSTN gene

Species specific primers and Taqman probes were designed and amplification conditions were optimized by using these primers and probes.

### Specificity of the Taqman probe systems.

The specificity of the 6 systems were tested for 8 common and commercial meat 100%) pure species (pork, beef, horse, sheep, chicken, turkey, rabbit, duck). No cross-reaction was obtained with any of the non target species DNA (table 2).

**Table 2 : Specificity and sensitivity test results obtained with the species specific primers-probe sets.**

| | | **Average Cp value ±SD** | | | | | |
|---|---|---|---|---|---|---|---|
| **species** | **DNA concentration (ng)** | **pork** | **beef** | **sheep** | **horse** | **turkey** | **chicken** |
| target species | 10 | 23,57 ± 0,11 | 23,99 ± 0,39 | 23,5 ± 0,05 | 24,14 ± 0,43 | 22,69 ± 0,15 | 23,34 ± 0,48 |
| | 3,3 | 25.16 ± 0,06 | 25,66 ± 0,19 | 25,06 ± 0,08 | 24,59 ± 0,62 | 24,29 ± 0,22 | 24,49 ± 0,51 |
| | 1,1 | 26,86 ± 0,02 | 27,28 ± 0,4 | 26,78 ± 0,05 | 26,99 ± 0,15 | 25,96 ± 0,15 | 26,08 ± 0,47 |
| | 0,36 | 28,47 ± 0,12 | 28,8 ± 0,25 | 28,45 ± 0,23 | 28,99 ± 0,64 | 28,07 ± 0,74 | 27,98 ± 0,19 |
| | 0,12 | 30,08 ± 0,03 | 30,44 ± 0,32 | 29,93 ± 0,11 | 30,0 ± 0,31 | 29,29 ± 0,11 | 29,73 ± 0,06 |
| | 0,04 | ± | 31,8 ± 0,14 | 31,4 ± 0,58 | 32,12 ± 0,61 | 30,94 ± 0,16 | 31,14 ± 0,2 |
| qPCR efficiency | | 1,937 | 1,968 | 1,886 | 1,991 | 1,963 | 1,99 |
| chicken | 10 | ND | ND | ND | ND | ND | 23,231 ± 0,4 |
| turkey | 10 | ND | ND | ND | ND | 22,72 ± 0,13 | ND |
| horse | 10 | ND | ND | ND | 24,04 ± 0,37 | ND | ND |
| donkey | 10 | ND | ND | ND | ND | ND | ND |
| pork | 10 | 23,39 ± 0,41 | ND | ND | ND | ND | ND |
| beef | 10 | ND | 24,01 ± 0,32 | ND | ND | ND | ND |
| sheep | 10 | ND | ND | 23,46 ± 0,082 | ND | ND | ND |
| rabbit | 10 | ND | ND | ND | ND | ND | ND |
| duck | 10 | ND | ND | ND | ND | ND | ND |

| | | | | | | | |
|---|---|---|---|---|---|---|---|
| ND = not detected. | | | | | | | |

### Sensitivity and linearity of Taqman probe systems.

The sensitivity and linearity of the 6 Taqman systems were tested by using a 3 -fold dilutions series of the genomic DNA obtained from each target species starting with lOng target DNA. The standard curve was generated by plotting the resulting Cp value compared with the logarithm of the target DNA concentration to test linearity. These linear regression were made by the LC480 software using the own Roche algorithms. The efficiency values (provided by the Roche algorithms) for the pork ; beef ; sheep ; horse ; turkey and chicken systems were respectively 1,937 ; 1,968 ; 1,886 ; 1,991 ; 1,963 and 1,99. As is well known, an acceptable range to determine the efficiency of a qPCR reaction and thus the linear relationship between the Cp value and the DNA concentration is between 1,8 and 2,2 (Efficiency= -1+10^{(-1/slope)}; 'a perfect efficiency= 2).

### Normalization methods

Sources of variability could be diversified (the nature and amount of starting sample, the DNA isolation process or lastly real-time PCR amplification). Normalization is essentially the act of neutralizing the effects of variability which could prevent the ability to compare data and lead to erroneous conclusions. The use of a normalizer gene is the most thorough method of addressing almost every source of variability in real-time PCR. In this study we have decided to use as normalizer a highly conserved region from myostatin fragment amplified (fig.2). This normalizer should have a similar amplification efficiency because we use the similar primers to amplify it.

To quantify data we could use comparative quantification and the ΔΔCₜ method. With this method Cₜs for the gene of interest in both the test sample and calibrator sample (here we use a 100% pure target species) are adjusted in relation to the normalizer gene Ct from the same 2 samples (ΔCₜ sample-ΔC_{t calibrator}= ΔΔCₜ). The resulting ΔΔCₜ value is incorporated to determine the fold difference in expression (fold difference= 2-^{ΔΔC}ₜ).

The invention is not limited by the Examples and the skilled person will appreciate alternatives and modifications that might be made to any step or component as herein described. The invention is therefore limited only by the scope of the claims.

### Results

### Metagenomic analysis using MSTN gene

The metagenomic assay was applied to 20 different DNA mixes including 8 different mammals and poultry DNA (beef, pork, horse, sheep, rabbit, goat, turkey and chicken), 13 mixes composed of 2 species at 50% of each DNA and 7 mixes composed of 4 species DNA at 25% of DNA.
The DNA of each species were prepared and eluted to the same concentration before mixing them. Because of differences in genome size, 100 ng of DNA will contain different amount of the common myostatin target gene from one species to another one explaining why in a 50-50 % mixture, small genome species (poultry, rabbit,..) will be overestimated compared with bigger genome species like mammals.

For example, because the genome size of poultry are twice as small as mammals genome, 100 ng of DNA will correspond to 3,5 10³ copy numbers of beef genomes compared to 9 10³ copy numbers of the chicken genomes). Thus at same concentration, chicken proportion are twice as much as mammals proportion.

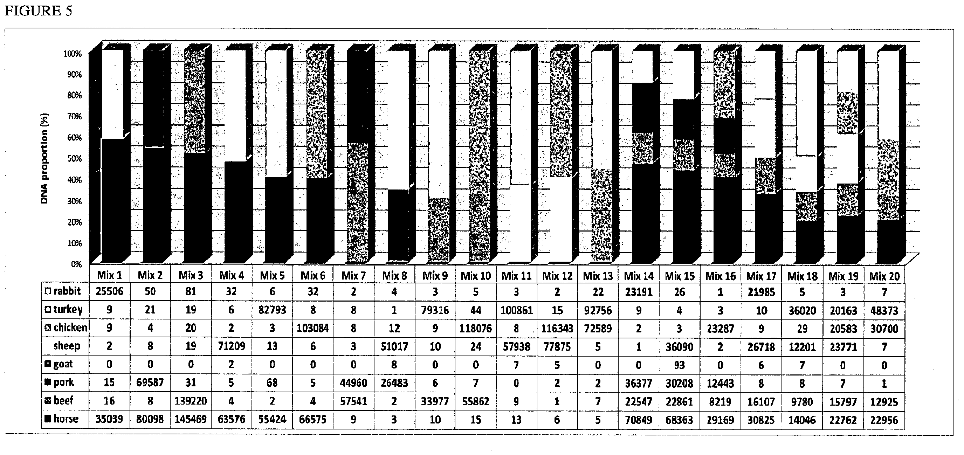

The treated sequences were compared to a private database using the Basic Local Alignment Search Tool. All the 8 species are correctly identified in the 20 samples tested. The proportions of each are almost representative taking into account the genome size (fig 5).

### Metagenomic analysis using ribosomal 12S rRNA.

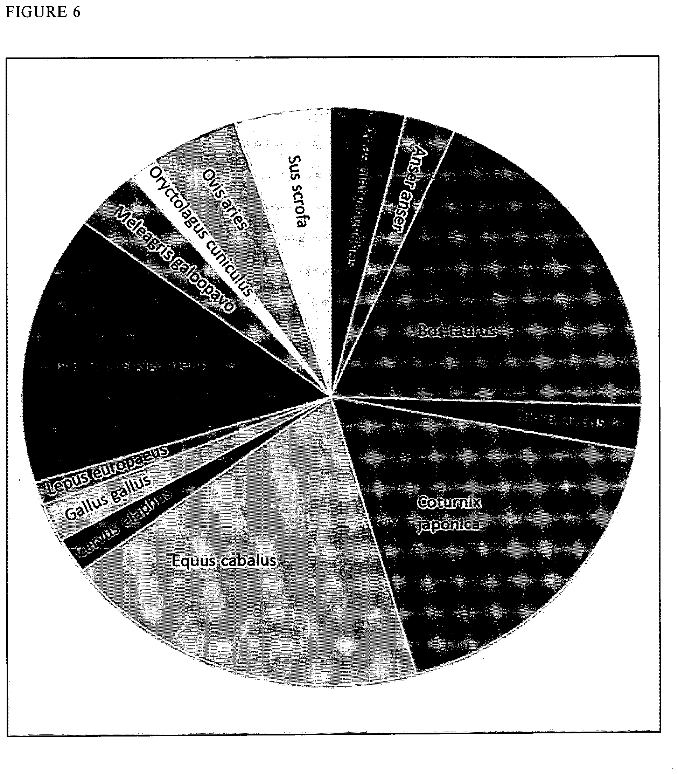

The metagenomic assay was applied to a mix of 14 mammal and poultry DNA all added at the same concentration (*Sus scrofa, Anser anser, Gallus gallus, Bos Taurus, Ovis aries, Equus caballus, Meleagris gallopavos, Capra hircus, Antilocapra Americana, Cervus elaphus, Anas platyrhyncho and Coturnix japonica* (fig 6). The treated sequences were compared to the NCBI database using the Basic Local Alignment Search Tool. The metagenomic analysis allowed the identification of all the 14 components but the proportions are not representative (depend on the meat tissues used and the mitochondria content of each).

## Claims

1. A method for determining the species origin(s) of DNA in a food sample comprising conducting metagenomic sequence analysis of the food sample by high throughput sequencing to identify DNA from at least one species, wherein the metagenomic analysis comprises amplification and sequencing of a metagenomic target DNA sequence which is part of the myostatin (MSTN) gene, said metagenomic target DNA sequence being selected to allow for: i) co-amplification of the metagenomic target DNA sequence from a plurality of species using a set of conserved, common primers comprising primers comprising or consisting of the sequence of SEQ ID NO:1,SEQ ID NO:2, and SEQ ID NO:3; and ii) the identification of species origin of any amplified metagenomic target DNA on the basis of its sequence; wherein the method comprises the step of amplifying target DNA using a set of primers comprising or consisting of the sequence of SEQ ID NO:1, SEQ ID NO:2, and SEQ ID NO:3.

2. The method of claim 1 further comprising species specific quantitative polymerase chain reaction (qPCR) to amplify and quantify a species specific target from the identified species DNA; and
optionally wherein the qPCR step is selected from qPCR using an intercalating dye and qPCR using a labelled reporter primer or probe; and
optionally wherein the labelled reporter primer or probe is part of a fluorescence/quencher reporter system or other fluorescence reporter system in which amplification of the species specific DNA target results in a reduction or elimination of fluorescence quenching, or other increase in fluorescence, to result in a detectable fluorescent signal; and
optionally wherein the reporter system comprises a hydrolysis probe, a molecular beacon probe, a pair of dual hybridization probes, an amplifluor primer system, a scorpion primer system, a light-up on- extension system or a QZyme primer system.

3. A method of any one of the preceding claims wherein the metagenomic target sequence has the same number of copies per genome in each of the species suspected as being present in the food sample, so as to further allow for a determination of the relative proportions of the different identified DNAs.

4. A method according to any one of claims 2-3 wherein the species specific target DNA sequence is part of the MSTN gene.

5. A method of any preceding claim wherein the metagenomic target DNA sequence is sequenced by a process for massively parallel sequencing; and
optionally wherein the process for massively parallel sequencing is selected from emulsion sequencing, and solid phase sequencing.

6. A kit for metagenomic identification of DNA present in a food sample, comprising a set of primers for the metagenomic amplification of a metagenomic target sequence in the myostatin gene, said metagenomic target sequence being selected to allow for: i) co- amplification of the metagenomic target DNA sequence from a plurality of species using a set of conserved, common primers of the kit comprising primers comprising or consisting of the sequence of SEQ ID NO. 1,SEQ ID NO. 2 and SEQ ID NO. 3; and ii) the identification of species origin of any amplified metagenomic target DNA on the basis of its sequence.

7. A kit of claim 6 wherein the metagenomic target sequence has the same number of copies per genome in each of the species suspected as being present in the food sample, so as to further allow for a determination of the relative proportions of the different identified DNAs; and/or
wherein the metagenomic target sequence is selected to allow for amplification of DNA from at least 12 or 13 different species using a set of conserved, common primers.

8. A method of claim 4 or the kit of claim 7 wherein at least one of the primers is modified by the addition of an adaptor sequence, tag or universal sequence.

9. A kit of claim 6 for quantitative PCR analysis of a species specific target sequence in a food sample, said kit comprising a further set of primers and probes for the amplification and real-time detection of a sequence from the myostatin gene, said sequence being selected to allow for species specific amplification and/or detection.

10. A method of any of claims 2 to 5 for the identification and quantification of at least one species specific target sequence in a food sample from any one or more of a species from a genus selected from *Sus, Gallus, Bos, Ovis, Equus, Meleagris, Felis, Capra, Antilocapra, Cervus, Anas, Anser and Coturnix, for example, Sus scrofa, Gallus gallus, Bos taurus, Ovis aries, Equus caballus, Meleagris gallopavo, Felis catus, Capra hircus, Antilocapra americana, Cervus elaphus, Anas platyrhyncho, Anser anser* and *Coturnix chinensis;* or
a kit of claim 9 wherein the species specific amplification and/or detection can distinguish between species from a genus selected from *Sus, Gallus, Bos, Ovis, Equus, Meleagris, Felis, Capra, Antilocapra, Cervus, Ana, Anser and Coturnix, for example, Sus scrofa, Gallus gallus, Bos taurus, Ovis aries, Equus caballus, Meleagris gallopavo, Felis catus, Capra hircus, Antilocapra americana, Cervus elaphus, Anas platyrhyncho, Anser anser* and *Coturnix chinensis.*

11. A method of any of claims 2 to 5 wherein the species specific target DNA sequence is amplified by a set of primers and the resulting amplicon is detected by a labelled hybridisation probe, or a kit of claim 9 wherein the set of primers and probes is selected from at least one of:
a) primers comprising or consisting of the sequences of SEQ ID NOS. 17 and 18 and a probe comprising or consisting of the sequence of SEQ ID NO. 19 for the quantitation of DNA sequences from pork (*Sus scrofa*); or
b) primers comprising or consisting of the sequences of SEQ ID NOS. 20 and 16 and a probe comprising or consisting of the sequence of SEQ ID NO. 21 for the quantitation of DNA sequences from chicken (*Gallus gallus*); or
c) primers comprising or consisting of the sequences of SEQ ID NOS. 22 and 16 and a probe comprising or consisting of the sequence of SEQ ID NO. 23 for the quantitation of DNA sequences from beef (*Bos taurus*); or
d) primers comprising or consisting of the sequences of SEQ ID NOS. 24 and 16 and a probe comprising or consisting of the sequence of SEQ ID NO. 25 for the quantitation of DNA sequences from sheep (*Ovis aries*); or
e) primers comprising or consisting of the sequences of SEQ ID NOS. 26 and 27 and a probe comprising or consisting of the sequence of SEQ ID NO. 28 for the quantitation of DNA sequences from horse (*Equus caballus*); or
f) primers comprising or consisting of the sequences of SEQ ID NOS. 29 and 16 and a probe comprising or consisting of the sequence of SEQ ID NO. 30 for the quantitation of DNA sequences from turkey (*Meleagris gallopavo*); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample; and
optionally wherein any one or more of primers having of SEQ ID NOs. 20, 22, 24 or 29 are replaced by a mixture of primers comprising or consisting of the sequences of SEQ ID NOs. 14 and 15.

12. A kit of any of claims 6 to 11 further comprising any reagent suitable for use in the metagenomic or qPCR analysis of the DNA content of a food sample.

13. The use of the primers as defined in claim 1 together with the primers and probes of claim 11 for the sequential metagenomic identification and species specific quantitation of DNA in a food sample, the selection of species specific primers and probes employed for quantitation being chosen on the basis of the metagenomic identification of species DNA present in the food sample; and optionally wherein the food sample is a meat sample.

## Patentansprüche

1. Verfahren zum Bestimmen der Speziesherkunft von DNA in einer Lebensmittelprobe, umfassend Durchführen einer metagenomischen Sequenzanalyse der Lebensmittelprobe mittels Hochdurchsatz-Sequenzierung zum Identifizieren von DNA von mindestens einer Spezies, wobei die metagenomische Analyse die Amplifikation und Sequenzierung einer metagenomischen DNA-Zielsequenz umfasst, die Teil des Myostatin-Gens (MSTN) ist, wobei die metagenomische DNA-Zielsequenz ausgewählt ist zum Ermöglichen von: i) Koamplifikation der metagenomischen DNA-Zielsequenz aus einer Vielzahl von Spezies mittels eines Sets von konservierten, gängigen Primern, umfassend Primer, welche die Sequenz von SEQ ID NO: 1, SEQ ID NO: 2 und SEQ ID NO: 3 umfassen oder daraus bestehen; und ii) Identifizierung der Speziesherkunft jeder amplifizierten metagenomischen Ziel-DNA auf der Grundlage ihrer Sequenz; wobei das Verfahren den Schritt des Amplifizierens von Ziel-DNA mittels eines Sets von Primern umfasst, welche die Sequenz von SEQ ID NO: 1, SEQ ID NO: 2 und SEQ ID NO: 3 umfassen oder daraus bestehen.

2. Verfahren nach Anspruch 1, weiter umfassend speziesspezifische quantitative Polymerase-Kettenreaktion (qPCR) zum Amplifizieren und Quantifizieren eines speziesspezifischen Ziels aus der identifizierten Spezies-DNA; und
wobei der qPCR-Schritt optional aus einer qPCR, die einen interkalierenden Farbstoff nutzt, und einer qPCR, die einen markierten Primer oder eine markierte Sonde als Reporter nutzt, ausgewählt ist; und
wobei der markierte Primer oder die markierte Sonde als Reporter optional Teil eines Fluoreszenz-Quencher-Reportersystems oder eines anderen Fluoreszenz-Reportersystems ist, in dem die Amplifikation der speziesspezifischen Ziel-DNA zu einer Verringerung oder einem Entfallen des Fluoreszenz-Quenchings oder einem anderen Anstieg der Fluoreszenz führt, um ein nachweisbares Fluoreszenzsignal zu ergeben; und
wobei das Reportersystem optional eine Hydrolysesonde, eine Molecular-Beacon-Sonde, ein Paar dualer Hybridisierungssonden, ein Amplifluor-Primersystem, ein Scorpion-Primersystem, ein Light-upon-Extension-System oder ein QZyme-Primersystem umfasst.

3. Verfahren nach einem der vorstehenden Ansprüche, wobei die metagenomische Zielsequenz dieselbe Anzahl von Kopien pro Genom in jeder der Spezies hat, von denen angenommen wird, dass sie in der Lebensmittelprobe vorhanden sind, um weiter eine Bestimmung der relativen Anteile der unterschiedlichen identifizierten DNAs zu ermöglichen.

4. Verfahren nach einem der Ansprüche 2-3, wobei die speziesspezifische DNA-Zielsequenz Teil des MSTN-Gens ist.

5. Verfahren nach einem der vorstehenden Ansprüche, wobei die metagenomische DNA-Zielsequenz mittels eines Verfahrens zur massiv parallelen Sequenzierung sequenziert wird; und
wobei das Verfahren zur massiv parallelen Sequenzierung optional aus Emulsionssequenzierung und Festphasensequenzierung ausgewählt ist.

6. Kit zur metagenomischen Identifizierung von in einer Lebensmittelprobe vorhandener DNA, umfassend ein Set von Primern für die metagenomische Amplifikation einer metagenomischen Zielsequenz im Myostatin-Gen, wobei die metagenomische Zielsequenz ausgewählt ist zum Ermöglichen von: i) Koamplifikation der metagenomischen DNA-Zielsequenz aus einer Vielzahl von Spezies mittels eines Sets von konservierten, gängigen Primern des Kits, umfassend Primer, welche die Sequenz von SEQ ID NO. 1, SEQ ID NO. 2 und SEQ ID NO. 3 umfassen oder daraus bestehen; und ii) Identifizierung der Speziesherkunft jeder amplifizierten metagenomischen Ziel-DNA auf der Grundlage ihrer Sequenz.

7. Kit nach Anspruch 6, wobei die metagenomische Zielsequenz dieselbe Anzahl von Kopien pro Genom in jeder der Spezies hat, von denen angenommen wird, dass sie in der Lebensmittelprobe vorhanden sind, um weiter eine Bestimmung der relativen Anteile der unterschiedlichen identifizierten DNAs zu ermöglichen; und/oder
wobei die metagenomische Zielsequenz ausgewählt ist zum Ermöglichen einer Amplifikation von DNA aus mindestens 12 oder 13 unterschiedlichen Spezies mittels eines Sets von konservierten, gängigen Primern.

8. Verfahren nach Anspruch 4 oder Kit nach Anspruch 7, wobei mindestens einer der Primer durch die Zugabe einer Adaptorsequenz, eines Tags oder einer universellen Sequenz modifiziert ist.

9. Kit nach Anspruch 6 für die quantitative PCR-Analyse einer speziesspezifischen Zielsequenz in einer Lebensmittelprobe, wobei das Kit ein weiteres Set von Primern und Sonden für die Amplifikation und den Echtzeit-Nachweis einer Sequenz aus dem Myostatin-Gen umfasst, wobei die Sequenz ausgewählt ist zum Ermöglichen von speziesspezifischer Amplifikation und/oder speziesspezifischem Nachweis.

10. Verfahren nach einem der Ansprüche 2 bis 5 für die Identifizierung und Quantifizierung von mindestens einer speziesspezifischen Zielsequenz in einer Lebensmittelprobe aus einer oder mehreren einer Spezies einer Gattung, ausgewählt aus *Sus, Gallus, Bos, Ovis, Equus, Meleagris, Felis, Capra, Antilocapra, Cervus, Anas, Anser und Coturnix, beispielsweise Sus scrofa, Gallus gallus, Bos taurus, Ovis aries, Equus caballus, Meleagris gallopavo, Felis catus, Capra hircus, Antilocapra americana, Cervus elaphus, Anas platyrhyncho, Anser anser und Coturnix chinensis;* oder
Kit nach Anspruch 9, wobei die speziesspezifische Amplifikation und/oder der speziesspezifische Nachweis zwischen Spezies aus einer Gattung unterscheiden kann, ausgewählt aus *Sus, Gallus, Bos, Ovis, Equus, Meleagris, Felis, Capra, Antilocapra, Cervus, Anas, Anser und Coturnix, beispielsweise Sus scrofa, Gallus gallus, Bos taurus, Ovis aries, Equus caballus, Meleagris gallopavo, Felis catus, Capra hircus, Antilocapra americana, Cervus elaphus, Anas platyrhyncho, Anser anser und Coturnix chinensis.*

11. Verfahren nach einem der Ansprüche 2 bis 5, wobei die speziesspezifische DNA-Zielsequenz mittels eines Sets von Primern amplifiziert wird und das erhaltene Amplikon durch eine markierte Hybridisierungssonde nachgewiesen wird, oder Kit nach Anspruch 9, wobei das Set von Primern und Sonden ausgewählt ist aus mindestens einem von:
a) Primern, welche die Sequenzen von SEQ ID NO. 17 und 18 umfassen oder daraus bestehen, und einer Sonde, welche die Sequenz von SEQ ID NO. 19 umfasst oder daraus besteht, für die Quantifizierung von DNA-Sequenzen aus Schweinefleisch (*Sus scrofa*); oder
b) Primern, welche die Sequenzen von SEQ ID NO. 20 und 16 umfassen oder daraus bestehen, und einer Sonde, welche die Sequenz von SEQ ID NO. 21 umfasst oder daraus besteht, für die Quantifizierung von DNA-Sequenzen aus Huhn (*Gallus gallus*); oder
c) Primern, welche die Sequenzen von SEQ ID NO. 22 und 16 umfassen oder daraus bestehen, und einer Sonde, welche die Sequenz von SEQ ID NO. 23 umfasst oder daraus besteht, für die Quantifizierung von DNA-Sequenzen aus Rindfleisch (*Bos taurus*); oder
d) Primern, welche die Sequenzen von SEQ ID NO. 24 und 16 umfassen oder daraus bestehen, und einer Sonde, welche die Sequenz von SEQ ID NO. 25 umfasst oder daraus besteht, für die Quantifizierung von DNA-Sequenzen aus Schaf (*Ovis aries*); oder
e) Primern, welche die Sequenzen von SEQ ID NO. 26 und 27 umfassen oder daraus bestehen, und einer Sonde, welche die Sequenz von SEQ ID NO. 28 umfasst oder daraus besteht, für die Quantifizierung von DNA-Sequenzen aus Pferd (*Equus caballus*); oder
f) Primern, welche die Sequenzen von SEQ ID NO. 29 und 16 umfassen oder daraus bestehen, und einer Sonde, welche die Sequenz von SEQ ID NO. 30 umfasst oder daraus besteht, für die Quantifizierung von DNA-Sequenzen aus Truthahn (*Meleagris gallopavo*); oder
g) Primern, welche die Sequenzen von SEQ ID NO. 14 und/oder 15 und 16 umfassen oder daraus bestehen, und einer Sonde, welche die Sequenz von SEQ ID NO. 31 oder 32 umfasst oder daraus besteht, für die Quantifizierung von DNA aller Spezies in der Probe; und
wobei optional ein oder mehrere Primer mit SEQ ID NO. 20, 22, 24 oder 29 durch ein Gemisch von Primern ersetzt werden, welche die Sequenzen von SEQ ID NO. 14 und 15 umfassen oder daraus bestehen.

12. Kit nach einem der Ansprüche 6 bis 11,
weiter umfassend ein beliebiges Reagenz, das für die Verwendung bei der metagenomischen Analyse oder qPCR-Analyse des DNA-Gehalts einer Lebensmittelprobe geeignet ist.

13. Verwendung der Primer nach Anspruch 1 zusammen mit den Primern und Sonden von Anspruch 11 für die sequenzielle metagenomische Identifizierung und speziesspezifische Quantifizierung von DNA in einer Lebensmittelprobe, wobei die Auswahl der speziesspezifischen Primer und Sonden, die für die Quantifizierung eingesetzt werden, auf der Grundlage der metagenomischen Identifizierung von Spezies-DNA getroffen wird, die in der Lebensmittelprobe vorhanden ist; und wobei die Lebensmittelprobe optional eine Fleischprobe ist.

## Revendications

1. Procédé de détermination de la ou des origine(s) d'espèces d'un ADN dans un échantillon alimentaire comprenant la réalisation d'une analyse de séquence métagénomique de l'échantillon alimentaire par séquençage à haut rendement pour identifier un ADN d'au moins une espèce, dans lequel l'analyse métagénomique comprend une amplification et un séquençage d'une séquence d'ADN cible métagénomique qui fait partie du gène de la myostatine (MSTN), ladite séquence d'ADN cible métagénomique étant sélectionnée pour permettre : i) une co-amplification de la séquence d'ADN cible métagénomique à partir d'une pluralité d'espèces en utilisant un ensemble d'amorces communes conservées comprenant des amorces comprenant ou consistant en la séquence de SEQ ID N° 1, SEQ ID N° 2, et SEQ ID N° 3; et ii) l'identification de l'origine d'une espèce d'un ADN cible métagénomique amplifié sur la base de sa séquence ; dans lequel le procédé comprend l'étape d'amplification d'un ADN cible en utilisant un ensemble d'amorces comprenant ou consistant en la séquence de SEQ ID N° 1, SEQ ID N° 2, et SEQ ID N° 3.

2. Procédé selon la revendication 1 comprenant en outre une réaction en chaîne polymérase quantitative spécifique de l'espèce (qPCR) pour amplifier et quantifier une cible spécifique d'une espèce à partir de l'ADN de l'espèce identifiée ; et
éventuellement dans lequel l'étape de qPCR est sélectionnée parmi une qPCR utilisant un colorant intercalant et une qPCR utilisant une amorce ou une sonde rapporteuse marquée ; et
éventuellement dans lequel l'amorce ou la sonde rapporteuse marquée fait partie d'un système rapporteur de fluorescence/ inactivation ou d'un autre système rapporteur de fluorescence dans lequel une amplification de l'ADN cible spécifique d'une espèce entraîne une réduction ou une élimination de l'inactivation de la fluorescence, ou une autre augmentation de la fluorescence, pour donner un signal fluorescent détectable ; et
éventuellement dans lequel le système rapporteur comprend une sonde d'hydrolyse, une sonde balise moléculaire, une paire de sondes d'hybridation double, un système d'amorce d'amplification, un système d'amorce scorpion, un système d'éclairage en extension ou un système d'amorce QZyme.

3. Procédé selon l'une quelconque des revendications précédentes, dans lequel la séquence cible métagénomique a le même nombre de copies par génome dans chacune des espèces suspectées d'être présentes dans l'échantillon alimentaire, de façon à permettre en outre une détermination des proportions relatives de différents ADN identifiés.

4. Procédé selon l'une quelconque des revendications 2 à 3, dans lequel la séquence d'ADN cible spécifique d'une espèce fait partie du gène MSTN.

5. Procédé selon l'une quelconque des revendications précédentes, dans lequel la séquence d'ADN cible métagénomique est séquencée par un procédé de séquençage parallèle massif; et
éventuellement dans lequel le procédé de séquençage parallèle massif est sélectionné parmi le séquençage en émulsion, et le séquençage en phase solide.

6. Trousse pour l'identification métagénomique d'un ADN présent dans un échantillon alimentaire, comprenant un ensemble d'amorces pour l'amplification métagénomique d'une séquence cible métagénomique dans le gène de la myostatine, ladite séquence cible métagénomique étant électionnée de façon à permettre : i) une co-amplification de la séquence d'ADN cible métagénomique à partir d'une pluralité d'espèces en utilisant un ensemble d'amorces communes conservées de la trousse comprenant des amorces comprenant ou consistant en la séquence de SEQ ID N° 1, SEQ ID N° 2, et SEQ ID N° 3; et ii) l'identification de l'origine d'une espèce d'un ADN cible métagénomique amplifié sur la base de sa séquence.

7. Trousse selon la revendication 6 dans laquelle la séquence cible métagénomique a le même nombre de copies par génome dans chacune des espèces suspectées d'être présentes dans l'échantillon alimentaire, de façon à permettre en outre une détermination des proportions relatives de différents ADN identifiés ; et/ou
dans laquelle la séquence métagénomique cible est sélectionnée de façon à permettre une amplification de l'ADN à partir d'au moins 12 ou 13 espèces différentes en utilisant un ensemble d'amorces communes conservées.

8. Procédé selon la revendication 4 ou trousse selon la revendication 7 dans lequel/laquelle au moins l'une des amorces est modifiée par l'addition d'une séquence d'adaptation, d'un marqueur ou d'une séquence universelle.

9. Trousse selon la revendication 6 pour une analyse par PCR quantitative d'une séquence cible spécifique d'une espèce dans un échantillon alimentaire, ladite trousse comprenant un ensemble supplémentaire d'amorces et de sondes pour l'amplification et la détection en temps réel d'une séquence du gène de la myostatine, ladite séquence étant sélectionnée pour permettre une amplification et/ou une détection spécifique d'une espèce.

10. Procédé selon l'une quelconque des revendications 2 à 5 pour l'identification et la quantification d'au moins une séquence cible spécifique d'une espèce dans un échantillon alimentaire parmi l'une quelconque ou plusieurs des espèces d'un genre sélectionné parmi *Sus, Gallus, Bos, Ovis, Equus, Meleagris, Felis, Capra, Antilocapra, Cervus, Anas, Anser* et *Coturnix,* par exemple, *Sus scrofa, Gallus gallus, Bos taurus, Ovis aries, Equus caballus, Meleagris gallopavo, Felis catus, Capra hircus, Antilocapra americana, Cervus elaphus, Anas platyrhyncho, Anser anser* et *Coturnix chinensis ;* ou
trousse selon la revendication 9 dans laquelle l'amplification et/ou la détection spécifique d'une espèce peut établir une distinction entre des espèces d'un genre sélectionné parmi *Sus, Gallus, Bos, Ovis, Equus, Meleagris, Felis, Capra, Antilocapra, Cervus, Anas, Anser* et *Coturnix,* par exemple, *Sus scrofa, Gallus gallus, Bos taurus, Ovis aries, Equus caballus, Meleagris gallopavo, Felis catus, Capra hircus, Antilocapra americana, Cervus elaphus, Anas platyrhyncho, Anser anser* et *Coturnix chinensis.*

11. Procédé selon l'une quelconque des revendications 2 à 5 dans lequel la séquence d'ADN cible spécifique d'une espèce est amplifiée par un ensemble d'amorces et l'amplicon obtenu est détecté par une sonde d'hybridation marquée, ou trousse selon la revendication 9 dans laquelle l'ensemble d'amorces et de sondes est sélectionné parmi au moins l'une parmi :
a) des amorces comprenant ou consistant en les séquences de SEQ ID N° 17 et 18 et une sonde comprenant ou consistant en la séquence de SEQ ID N° 19 pour la quantification de séquence d'ADN du porc (*Sus scrofa*) ; ou
b) des amorces comprenant ou consistant en les séquences de SEQ ID N° 20 et 16 et une sonde comprenant ou consistant en la séquence de SEQ ID N° 21 pour la quantification de séquence d'ADN du poulet (*Gallus gallus*) ; ou
c) des amorces comprenant ou consistant en les séquences de SEQ ID N° 22 et 16 et une sonde comprenant ou consistant en la séquence de SEQ ID N° 23 pour la quantification de séquence d'ADN du boeuf (*Bos taurus*) ; ou
d) des amorces comprenant ou consistant en les séquences de SEQ ID N° 24 et 16 et une sonde comprenant ou consistant en la séquence de SEQ ID N° 25 pour la quantification de séquence d'ADN du mouton (*Ovis aries*) ; ou
e) des amorces comprenant ou consistant en les séquences de SEQ ID N° 26 et 27 et une sonde comprenant ou consistant en la séquence de SEQ ID N° 28 pour la quantification de séquence d'ADN du cheval (*Equus caballus*) ; ou
f) des amorces comprenant ou consistant en les séquences de SEQ ID N° 29 et 16 et une sonde comprenant ou consistant en la séquence de SEQ ID N° 30 pour la quantification de séquence d'ADN de la dinde (*Meleagris gallopavo*) ; ou
g) des amorces comprenant ou consistant en les séquences de SEQ ID N° 14 et/ou 15 et 16 et une sonde comprenant ou consistant en la séquence de SEQ ID N° 31 ou 32 pour la quantification d'ADN de toutes les espèces dans l'échantillon ; et
éventuellement dans lequel l'une quelconque ou plusieurs des amorces ayant SEQ ID N° 20, 22, 24 ou 29 sont remplacées par un mélange d'amorces comprenant ou consistant en les séquences de SEQ ID N° 14 et 15.

12. Trousse selon l'une quelconque des revendications 6 à 11, comprenant en outre un réactif quelconque approprié pour une utilisation dans l'analyse métagénomique ou aanlyse par qPCR du contenu de l'ADN d'un échantillon alimentaire.

13. Utilisation des amorces telles que définies dans la revendication 1 conjointement avec les amorces et sondes selon la revendication 11 pour l'identification métagénomique séquentielle et la quantification d'ADN spécifique d'une espèce dans un échantillon alimentaire, la sélection d'amorces et sondes spécifiques d'une espèce employées pour la quantification étant réalisée sur la base de l'identification métagénomique d'un ADN d'espèce présent dans l'échantillon alimentaire; et éventuellement dans lequel l'échantillon alimentaire est un échantillon de viande.
